# EUROPEAN PATENT APPLICATION

(11) **EP 4 556 492 A1**
(43) Date of publication of application: **21.05.2025**
(21) Application number: 23306974.9
(22) Date of filing: 14.11.2023
(51) Int. Cl.: C07K 16/28, A61P 9/10

(54) **TREATMENT OF CARDIOVASCULAR DISEASES USING ANTI-HUMAN GPVI ANTIBODIES IN SUBPOPULATIONS OF SUBJECTS**

(71) Applicant: Acticor Biotech, 75014 Paris (FR)
(72) Inventor: The designation of the inventor has not yet been filed
(74) Representative: Icosa

(57) **Abstract**

The present invention relates to the treatment of cardiovascular diseases or events. In particular, the present invention relates to a method for treating a cardiovascular disease or event, in particular a stroke, comprising the administration of an inhibitor of the GPVI signaling pathway, in particular an anti-human glycoprotein VI antibody or fragment thereof.

## Description

### FIELD OF INVENTION

The present invention relates to the treatment of cardiovascular diseases or events. In particular, the present invention relates to a method for treating a cardiovascular disease or event, in particular a stroke, comprising the administration of an inhibitor of the GPVI signaling pathway, in particular an anti-human glycoprotein VI antibody or fragment thereof.

### BACKGROUND OF INVENTION

Acute coronary and cerebrovascular accidents are currently a major cause of death in the world. In addition, the global incidence of recurrence and death in the 6-month post-treatment period after an acute coronary syndrome is still 17%.

In the case of acute coronary syndrome with ST segment elevation, mechanical treatment with coronary angioplasty and introduction of a stent is highly efficient to urgently restore coronary artery flow, but does not prevent morbidity/mortality for about 15% of patients in the next 6 months. Thrombolytic treatments, which are based on long-term fibrinolytic, anticoagulant and anti-aggregating drugs associations, give even less encouraging results. Indeed, despite improvements in medical treatment of thrombosis, morbidity/mortality at 6 months is similar to that observed for acute coronary syndrome without ST segment elevation.

Regarding cerebrovascular ischemic accidents, treatments are still very limited due to the generally late caring of most patients and to the hemorrhagic risk of currently available anti-thrombotic treatments.

There is thus still a clinical need for improved treatments for cardiovascular diseases, and especially for new molecules with improved features compared to available molecules and for specific dosage regimens for administering said molecules. The challenge to face is to obtain molecules and protocols of administration with excellent efficiency on the pathological thrombosis but devoid of risk of bleeding.

Glycoprotein VI has been demonstrated in animals to play a role in experimental thrombosis including stroke, vascular remodeling and to be critical in atherothrombosis. Contrary to αIIbβ3 integrin antagonists, which are currently used in thrombosis treatment and inhibit platelet final activation phase, *i.e.,* platelet aggregation, and to antagonists of platelet recruitment (*e.g.,* inhibitors of the P2Y12 ADP receptor and aspirin), GPVI is implicated into several steps of the platelet plug formation: initiation via the interaction with the injured vascular wall, amplification via initial platelet activation leading to the secretion of secondary agonists, the activation of integrins and of platelet procoagulant activity, growth and stabilization via the interaction with fibrin (Mammadova-Bach et al., 2015. Blood. 126(5):683-91). Thus, it was hypothesized that GPVI antagonists may prevent not only platelet aggregation, but also secondary agonists liberation as well as growth factors and cytokines secretion resulting into vascular lesions development. Finally, GPVI expression is limited to platelets and megakaryocytes, and thus represents a perfectly specific target for anti-thrombosis treatment.

A humanized antibody fragment with GPVI antagonist activity was described in WO2017/021539. This antibody fragment, named ACT017 or glenzocimab, was demonstrated to be an agent that possess anti-platelet activity and efficient antithrombotic potential in models of thrombosis, without increasing bleeding.

The Inventors have surprisingly found that said antibody fragment is particularly efficient in subpopulations of subjects suffering from a stroke, and in particular, in subjects presenting a large ischemic core volume, and/or a good cerebral collateral circulation.

### SUMMARY

The present invention relates to an isolated humanized protein binding to human Glycoprotein VI (hGPVI) for use in treating a GPVI-related condition in a subject in need thereof, wherein said subject presents at least one of the following conditions:
i) said subject has a large ischemic core volume, and/or
ii) said subject has a good cerebral collateral circulation,
wherein the cerebral collateral circulation is assessed by either measuring the Tan Score with computed tomography angiography (CTA), or measuring the hypoperfusion intensity ratio (HIR) with CTP or magnetic resonance imaging perfusion (MRP).

The isolated humanized protein binding to hGPVIfor use as defined hereinabove, wherein the GPVI-related condition is a stroke, preferably an acute ischemic stroke.

The isolated humanized protein binding to hGPVIfor use as defined hereinabove, wherein the ischemic core volume is assessed by measuring the acute infarct volume (AIV) with magnetic resonance imaging (MRI), and/or computed tomography (CT), including CT perfusion (CTP), non-contrast computed tomography (NCCT) and computed tomography angiography (CTA) .

The isolated humanized protein binding to hGPVIfor use as defined hereinabove, wherein the subject with a large ischemic core volume has an ischemic core volume equals or above 7.0 mL.

The isolated humanized protein binding to hGPVIfor use as defined hereinabove, wherein the cerebral collateral circulation is assessed by either measuring the Tan Score with computed tomography angiography (CTA).

The isolated humanized protein binding to hGPVIfor use as defined hereinabove, wherein the subject with a good cerebral collateral circulation has a Tan score value of 2 or 3.

The isolated humanized protein binding to hGPVIfor use as defined hereinabove, wherein the protein protects the cerebral tissue following the GPVI-related condition.

The isolated humanized protein binding to hGPVI for use as defined hereinabove, wherein the protein decreases the volume of infarct, the volume of hemorrhagic transformation and/or the volume of ischemic injury following the GPVI-related condition.

The isolated humanized protein binding to hGPVI for use as defined hereinabove, being an antibody molecule or an antibody fragment selected from the group consisting of a whole antibody, a single chain variable fragment (scFv), a Fv fragment, a Fab fragment, a unibody, a domain antibody, and a nanobody; or a monomeric antibody mimetic selected from the group consisting of an affibody, an affilin, an affitin, an adnectin, an atrimer, an evasin, a DARPin, an anticalin, an avimer, a fynomer, and a versabody.

The isolated humanized protein binding to hGPVI for use as defined hereinabove, wherein the isolated humanized protein is a monovalent antibody or fragment thereof.

The isolated humanized protein binding to hGPVI for use as defined hereinabove, being an antibody molecule or an antibody fragment,
- wherein the variable region of the heavy chain comprises at least one of the following CDRs:
   VH-CDR1: GYTFTSYNMH (SEQ ID NO: 1);
   VH-CDR2: GIYPGNGDTSYNQKFQG (SEQ ID NO: 2); and
   VH-CDR3: GTVVGDWYFDV (SEQ ID NO: 3);
   or any CDR having an amino acid sequence that shares at least 60% of identity with SEQ ID NO: 1-3; and
- the variable region of the light chain comprises at least one of the following CDRs:
   VL-CDR1: RSSQSLENSNGNTYLN (SEQ ID NO: 4);
   VL-CDR2: RVSNRFS (SEQ ID NO: 5); and
   VL-CDR3: LQLTHVPWT (SEQ ID NO: 6);
   or any CDR having an amino acid sequence that shares at least 60% of identity with SEQ ID NO: 4-6.

The isolated humanized protein binding to hGPVI for use as defined hereinabove, being an antibody molecule or an antibody fragment, wherein the variable region of the heavy chain comprises the following CDRs: GYTFTSYNMH (SEQ ID NO: 1), GIYPGNGDTSYNQKFQG (SEQ ID NO: 2) and GTVVGDWYFDV (SEQ ID NO: 3) and the variable region of the light chain comprises the following CDRs: RSSQSLENSNGNTYLN (SEQ ID NO: 4), RVSNRFS (SEQ ID NO: 5) and LQLTHVPWT (SEQ ID NO: 6) or any CDR having an amino acid sequence that shares at least 60% of identity with said SEQ ID NO: 1-6.

The isolated humanized protein binding to hGPVI for use as defined hereinabove, being an antibody molecule or an antibody fragment, wherein the amino acid sequence of the heavy chain variable region is SEQ ID NO: 7 or any sequence having an amino acid sequence that shares at least 60% of identity with said SEQ ID NO: 7, and wherein the amino acid sequence of the light chain variable region is SEQ ID NO: 8, or any sequence having an amino acid sequence that shares at least 60% of identity with said SEQ ID NO: 8.

The isolated humanized protein binding to hGPVI for use as defined hereinabove, being an antibody molecule or an antibody fragment, wherein the amino acid sequence of the heavy chain variable region is SEQ ID NO: 7 or any sequence having an amino acid sequence that shares at least 60% of identity with said SEQ ID NO: 7, and wherein the amino acid sequence of the light chain variable region is SEQ ID NO: 9, or any sequence having an amino acid sequence that shares at least 60% of identity with said SEQ ID NO: 9.

### DEFINITIONS

In the present invention, the following terms have the following meanings:
"**Glycoprotein VI (GPVI)**" is a platelet membrane glycoprotein that is involved in platelet-collagen interactions. GPVI is a transmembrane collagen receptor expressed on the surface of platelets. In one embodiment, the amino acid sequence of human GPVI is SEQ ID NO: 13 (accession number: BAA89353.1) or any amino acid sequence presenting at least about 90% identity with SEQ ID NO: 13, preferably at least about 91, 92, 93, 94, 95, 96, 97, 98, 99% identity or more with SEQ ID NO: 13.

SEQ ID NO: 13
MSPSPTALFCLGLCLGRVPA (Signal peptide).
QSGPLPKPSLQALPSSLVPLEKPVTLRCQGPPGVDLYRLEKLSSSRYQDQAVLFI
PAMKRSLAGRYRCSYQNGSLWSLPSDQLELVATGVFAKPSLSAQPGPAVSSGG
DVTLQCQTRYGFDQFALYKEGDPAPYKNPERWYRASFPIITVTAAHSGTYRCY
SFSSRDPYLWSAPSDPLELVVTGTSVTPSRLPTEPPSSVAEFSEATAELTVSFTNK
VFTTETSRSITTSPKESDSPAGPARQYYTKGN (Extracellular domain).
LVRICLGAVILIILAGFLAEDWHSRRKRLRHRGRAVQRPLPPLPPLPQTRKSHGG
QDGGRQDVHSRGLCS (Transmembrane and cytoplasmic domains).

The extracellular domain of GPVI is composed of two Ig-like C2-type domains, namely D1 and D2, linked by a hinge interdomain. In one embodiment, D1 comprises amino acid residues 21 to 109 of SEQ ID NO: 13. In one embodiment, the hinge interdomain between D1 and D2 comprises amino acid residues 110 to 113 of SEQ ID NO: 13. In one embodiment, D2 comprises amino acid residues 114 to 207 of SEQ ID NO: 13.

"**About**" preceding a figure means plus or less 10% of the value of said figure.

"**Antibody**" or **"Immunoglobulin"** - As used herein, the term "immunoglobulin" includes a protein having a combination of two heavy and two light chains whether or not it possesses any relevant specific immunoreactivity. "Antibodies" refers to such assemblies which have significant known specific immunoreactive activity to an antigen of interest (*e.g.* human GPVI). The term "anti-GPVI antibodies" is used herein to refer to antibodies which exhibit immunological specificity for human GPVI protein. As explained elsewhere herein, "specificity" for human GPVI does not exclude crossreaction with species homologues of GPVI. Antibodies and immunoglobulins comprise light and heavy chains, with or without an interchain covalent linkage between them. Basic immunoglobulin structures in vertebrate systems are relatively well understood. The generic term "immunoglobulin" comprises five distinct classes of antibody that can be distinguished biochemically. All five classes of antibodies are within the scope of the present invention; the following discussion will generally be directed to the IgG class of immunoglobulin molecules. The light chains of an antibody are classified as either kappa or lambda ([κ], [λ]). Each heavy chain class may be bonded with either a kappa or lambda light chain. Those skilled in the art will appreciate that heavy chains are classified as gamma, mu, alpha, delta, or epsilon (γ, µ, α, 8, ε) with some subclasses among them (*e.g.,* γ1 - γ4). It is the nature of this chain that determines the "class" of the antibody as IgG, IgM, IgA IgD, or IgE, respectively. The immunoglobulin subclasses (isotypes) *e.g.,* IgGl, IgG2, IgG3, IgG4, IgA1, etc. are well characterized and are known to confer functional specialization. As indicated above, the variable region of an antibody allows the antibody to selectively recognize and specifically bind epitopes on antigens. That is, the light chain variable domain (VL domain) and heavy chain variable domain (VH domain) of an antibody combine to form the variable region that defines a three-dimensional antigen binding site. This quaternary antibody structure forms the antigen binding site presents at the end of each arm of the "Y". More specifically, the antigen binding site is defined by three complementarity determining regions (CDRs) on each of the VH and VL chains.

"**An isolated protein"** or **"an isolated antibody"** - As used herein, an "isolated protein", in particular an "isolated antibody", is one that has been separated and/or recovered from a component of its natural environment. Contaminant components of its natural environment are materials that would interfere with diagnostic or therapeutic uses of the antibody, and may include enzymes, hormones, and other proteinaceous or nonproteinaceous components. In preferred embodiments, the protein, in particular the antibody, is purified: (1) to greater than 80, 85, 90, 91, 92, 93, 94, 95% or more by weight of antibody as determined by the Lowry method, and most preferably more than 99% by weight; (2) to a degree sufficient to obtain at least 15 residues of N-terminal or internal amino acid sequence by use of a spinning cup sequenator; or (3) to homogeneity as shown by SDS-PAGE under reducing or non-reducing conditions and using Coomassie blue or, preferably, silver staining. Isolated protein, in particular isolated antibody, includes the protein or antibody in situ within recombinant cells since at least one component of the protein's or antibody's natural environment will not be present. Ordinarily, however, isolated protein, in particular isolated antibody, will be prepared by at least one purification step.

**"Binding Site"** - As used herein, the term "binding site" comprises a region of a protein which is responsible for selectively binding to a target antigen of interest (*e.g.* human GPVI). Binding domains or binding regions comprise at least one binding site. Exemplary binding domains include an antibody variable domain. The protein of the invention may comprise a single antigen binding site or multiple (*e.g.,* two, three or four) antigen binding sites. Preferably, however, the protein of the invention comprises a single antigen binding site.

**"Conservative amino acid substitution"** - As used herein, "conservative amino acid substitutions" are ones in which the amino acid residue is replaced with an amino acid residue that has similar properties, such that one skilled in the art of peptide chemistry would expect the secondary structure and hydropathic nature of the polypeptide to be substantially unchanged. Amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine. Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. Other families of amino acid residues having similar side chains have been defined in the art, including basic side chains *(e.g.,* lysine, arginine, histidine), acidic side chains *(e.g.,* aspartic acid, glutamic acid), uncharged polar side chains (*e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Thus, a nonessential amino acid residue in an immunoglobulin polypeptide may be replaced with another amino acid residue from the same side chain family. In another embodiment, a string of amino acids can be replaced with a structurally similar string that differs in order and/or composition of side chain family members.

**"Chimeric"** - As used herein, a "chimeric" protein comprises a first amino acid sequence linked to a second amino acid sequence with which it is not naturally linked in nature. The amino acid sequences may normally exist in separate proteins that are brought together in the fusion protein or they may normally exist in the same protein but are placed in a new arrangement in the fusion protein. A chimeric protein may be created, for example, by chemical synthesis, or by creating and translating a polynucleotide in which the peptide regions are encoded in the desired relationship.

**"CDR"** - As used herein, the term "CDR" or "complementarity determining region" means the non-contiguous antigen combining sites found within the variable region of both heavy and light chain polypeptides. CDRs were identified according to the following rules as deduced from Kabat (Kabat *et al.,* Identical V region amino acid sequences and segments of sequences in antibodies of different specificities. Relative contributions of VH and VL genes, minigenes, and complementarity-determining regions to binding of antibody-combining sites, J Immunol. 1991 Sep 1;147(5):1709-19) and Chothia & Lesk (Chothia C. and A.M. Lesk, "Canonical structures for the hypervariable regions of immunoglobulins", J Mol Biol. 1987 Aug 20;196(4):901-17):
- CDR-L1:
   Start - Approx residue 24;
   Residue before is always a Cys;
   Residue after is always a Trp. Typically TRP-TYR-GLN, but also, TRP-LEU-GLN, TRP-PHE-GLN, TRP-TYR-LEU;
   Length 10 to 17 residues;
- CDR-L2:
   Start - always 16 residues after the end of L1;
   Residues before generally ILE-TYR, but also, VAL-TYR, ILE-LYS, ILE-PHE;
   Length always 7 residues;
- CDR-L3:
   Start - always 33 residues after end of L2;
   Residue before is always Cys;
   Residues after always PHE-GLY-XXX-GLY;
   Length 7 to 11 residues;
- CDR-H1:
   Start - Approx residue 26 (always 4 after a CYS) [Chothia / AbM definition] Kabat definition starts 5 residues later;
   Residues before always CYS-XXX-XXX-XXX;
   Residues after always a TRP. Typically TRP-VAL, but also, TRP-ILE, TRP-ALA
   Length 10 to 12 residues (AbM definition) Chothia definition excludes the last 4 residues;
- CDR-H2:
   Start - always 15 residues after the end of Kabat / AbM definition) of CDR-H1 Residues before typically LEU-GLU-TRP-ILE-GLY (SEQ ID NO: 21), but a number of variations;
   Residues after LYS/ARG-LEU/II,E/VAL/PHE/THR/ALA-THR/SER/II,E/ALA Length Kabat definition 16 to 19 residues (AbM definition ends 7 residues earlier);
- CDR-H3:
   Start - always 33 residues after end of CDR-H2 (always 2 after a CYS);
   Residues before always CYS-XXX-XXX (typically CYS-ALA-ARG);
   Residues after always TRP-GLY-XXX-GLY;
   Length 3 to 25 residues.

**"CH2 domain"** - As used herein, the term "CH2 domain" includes the region of a heavy chain molecule that usually extends from about amino acid 231 to about amino acid 340. The CH2 domain is unique in that it is not closely paired with another domain. Rather, two N-linked branched carbohydrate chains are interposed between the two CH2 domains of an intact native IgG molecule.

**"Derived from"** - As used herein, the term "derived from" a designated protein *(e.g.,* an anti-GPVI antibody or antigen-binding fragment thereof) refers to the origin of the protein. In an embodiment, the protein or amino acid sequence which is derived from a particular starting protein is a CDR sequence or sequence related thereto. In an embodiment, the amino acid sequence which is derived from a particular starting protein is not contiguous. For example, in an embodiment, one, two, three, four, five, or six CDRs are derived from a starting antibody. In an embodiment, the protein or amino acid sequence which is derived from a particular starting protein or amino acid sequence has an amino acid sequence that is essentially identical to that of the starting sequence, or a region thereof wherein the region consists of at least 3-5 amino acids, at least 5-10 amino acids, at least 10-20 amino acids, at least 20-30 amino acids, or at least 30-50 amino acids, or which is otherwise identifiable to one of ordinary skill in the art as having its origin in the starting sequence. In an embodiment, the one or more CDR sequences derived from the starting antibody are altered to produce variant CDR sequences, *e.g.,* affinity variants, wherein the variant CDR sequences maintain GPVI binding activity.

**"Diabodies"** - As used herein, the term "diabodies" refers to small antibody fragments prepared by constructing sFv fragments (see sFv paragraph) with short linkers (about 5-10 residues) between the VH and VL domains such that inter-chain but not intrachain pairing of the V domains is achieved, resulting in a bivalent fragment, *i.e.,* fragment having two antigen-binding sites. Bispecific diabodies are heterodimers of two "crossover" sFv fragments in which the VH and VL domains of the two antibodies are present on different polypeptide chains.

**"Engineered"** - As used herein, the term "engineered" includes manipulation of nucleic acid or polypeptide molecules by synthetic means (*e.g.,* by recombinant techniques, *in vitro* peptide synthesis, by enzymatic or chemical coupling of peptides or some combination of these techniques). Preferably, the antibodies or fragments of the invention are engineered, including for example, humanized and/or chimeric antibodies, and antibodies or fragments which have been engineered to improve one or more properties, such as antigen binding, stability/half-life or effector function. In particular, antibodies or fragments thereof can be modified using known methods, for example to slow clearance *in vivo* and obtain a more desirable pharmacokinetic profile the fragment may be modified with polyethylene glycol (PEG).

**"Epitope"** - As used herein, the term "epitope" refers to a specific arrangement of amino acids located on a protein or proteins to which an antibody binds. Epitopes often consist of a chemically active surface grouping of molecules such as amino acids or sugar side chains, and have specific three dimensional structural characteristics as well as specific charge characteristics. Epitopes can be linear or conformational, *i.e.,* involving two or more sequences of amino acids in various regions of the antigen that may not necessarily be contiguous.

**"Framework region"** - As used herein, the term "framework region" or "FR region" includes the amino acid residues that are part of the variable region, but are not part of the CDRs (*e.g.,* using the Kabat/Chothia definition of CDRs). Therefore, a variable region framework is between about 100-120 amino acids in length but includes only those amino acids outside of the CDRs. For the specific example of a heavy chain variable region and for the CDRs as defined by Kabat/Chothia, framework region 1 may correspond to the domain of the variable region encompassing amino acids 1-25; framework region 2 may correspond to the domain of the variable region encompassing amino acids 36-49; framework region 3 may correspond to the domain of the variable region encompassing amino acids 67-98, and framework region 4 may correspond to the domain of the variable region from amino acids 110 to the end of the variable region. The framework regions for the light chain are similarly separated by each of the light chain variable region CDRs. The antigen binding site formed by the positioned CDRs defines a surface complementary to the epitope on the immunoreactive antigen. This complementary surface promotes the non-covalent binding of the antibody to the immunoreactive antigen epitope. The position of CDRs can be readily identified by one of ordinary skill in the art.

**"Fragment"** - As used herein, the term "fragment" refers to a part or region of an antibody or antibody chain comprising fewer amino acid residues than an intact or complete antibody or antibody chain. The term "antigen-binding fragment" refers to a protein fragment of an immunoglobulin or antibody that binds antigen or competes with intact antibody (*i.e.,* with the intact antibody from which they were derived) for antigen binding (*i.e.,* specific binding to human GPVI). As used herein, the term "fragment" of an antibody molecule includes antigen-binding fragments of antibodies, for example, an antibody light chain variable domain (VL), an antibody heavy chain variable domain (VH), a single chain variable fragment (scFv), a single chain antibody, a dimeric single chain antibody, a Fab fragment, a Fab' fragment, a Fab'-SH fragment, a F(ab')₂ fragment, a Fd fragment, a Fv fragment, a single domain antibody fragment (DAb), a one-armed (monovalent) antibody, a domain antibody, a unibody, a nanobody, a diabody, a triabody, a tetrabody, or any antigen-binding molecule formed by combination, assembly or conjugation of such antigen binding fragments. The term "fragment" includes single chain proteins containing only one light chain variable domain, or a fragment thereof that contains the three CDRs of the light chain variable domain, without an associated heavy chain moiety and single chain proteins containing only one heavy chain variable region, or a fragment thereof containing the three CDRs of the heavy chain variable region, without an associated light chain moiety. Fragments can be obtained, *e.g.,* via chemical or enzymatic treatment of an intact or complete antibody or antibody chain or by recombinant means. For instance, Fab or F(ab')2 fragments may be produced by protease digestion of the isolated antibodies, according to conventional techniques.

"**Fc fragment"** - As used herein, the term "Fc fragment" of an antibody comprises the carboxy-terminal portions of both H chains held together by disulfides. The effector functions of antibodies are determined by sequences in the Fc region, which region is also the part recognized by Fc receptors (FcR) found on certain types of cells.

**"Fv"** - As used herein, the term "Fv" is the minimum antibody fragment that contains a complete antigen-recognition and -binding site. This fragment consists of a dimer of one heavy- and one light-chain variable region domain in tight, non-covalent association. From the folding of these two domains emanate six hypervariable loops (three loops each from the H and L chain) that contribute to antigen binding and confer antigen binding specificity to the antibody. However, even a single variable domain (or half of an Fv comprising only three CDRs specific for an antigen) has the ability to recognize and bind antigen, although at a lower affinity than the entire binding site.

"**Heavy chain region"** - As used herein, the term "heavy chain region" includes amino acid sequences derived from the constant domains of an immunoglobulin heavy chain. A protein comprising a heavy chain region comprises at least one of: a CH1 domain, a hinge (*e.g.,* upper, middle, and/or lower hinge region) domain, a CH2 domain, a CH3 domain, or a variant or fragment thereof. In certain embodiments, the constituent constant domains of the heavy chain region are from different immunoglobulin molecules. For example, a heavy chain region of a protein may comprise a CH2 domain derived from an IgG1 molecule and a hinge region derived from an IgG3 or IgG4 molecule. In other embodiments, the constant domains are chimeric domains comprising regions of different immunoglobulin molecules. For example, a hinge may comprise a first region from an IgG1 molecule and a second region from an IgG3 or IgG4 molecule. As set forth above, it will be understood by one of ordinary skill in the art that the constant domains of the heavy chain region may be modified such that they vary in amino acid sequence from the naturally occurring (wild-type) immunoglobulin molecule. That is, the proteins of the invention disclosed herein may comprise alterations or modifications to one or more of the heavy chain constant domains (CH1, hinge, CH2 or CH3) and/or to the light chain constant domain (CL). Exemplary modifications include additions, deletions or substitutions of one or more amino acids in one or more domains.

**"Hinge region"** - As used herein, the term "hinge region" includes the region of a heavy chain molecule that joins the CH1 domain to the CH2 domain. This hinge region comprises approximately 25 residues and is flexible, thus allowing the two N-terminal antigen binding regions to move independently.

The terms **"hypervariable loop"** and **"complementarity determining region"** are not strictly synonymous, since the hypervariable loops (HVs) are defined on the basis of structure, whereas complementarity determining regions (CDRs) are defined based on sequence variability and the limits of the HVs and the CDRs may be different in some VH and VL domains. The CDRs of the VL and VH domains can typically be defined by the Kabat/Chothia definition (see above). The HVs may be comprised within the corresponding CDRs and references herein to the "hypervariable loops" of VH and VL domains should be interpreted as also encompassing the corresponding CDRs, and vice versa, unless otherwise indicated. The more highly conserved regions of variable domains are called the framework region (FR), as defined herein.

**"Humanized"** - As used herein, the term "humanized" refers to chimeric immunoglobulins, immunoglobulin chains or fragments thereof (such as Fv, Fab, Fab', or other antigen-binding subsequences of antibodies) which contain minimal sequence derived from a murine immunoglobulin. For example, humanized antibodies are human immunoglobulins (recipient antibody) in which residues from a complementarydetermining region (CDR) of the recipient are replaced by residues from a CDR of the original antibody (donor antibody) while maintaining the desired specificity, affinity, and capacity of the original antibody.

**"Humanizing substitutions"** - As used herein, the term "humanizing substitutions" refers to amino acid substitutions in which the amino acid residue present at a particular position in the VH or VL domain of a non-human anti-GPVI antibody (for example a murine anti-GPVI antibody) is replaced with an amino acid residue which occurs at an equivalent position in a reference human VH or VL domain. The reference human VH or VL domain may be a VH or VL domain encoded by the human germline, in which case the substituted residues may be referred to as "germlining substitutions". Humanizing/germlining substitutions may be made in the framework regions and/or the CDRs of an anti-GPVI antibody, defined herein.

**"High human homology"** - An antibody comprising a heavy chain variable domain (VH) and a light chain variable domain (VL) will be considered as having high human homology if the VH domains and the VL domains, taken together, exhibit at least 70, 75, 80, 85, 90, 95% or more amino acid sequence identity to the closest matching human germline VH and VL sequences. Antibodies having high human homology may include antibodies comprising VH and VL domains of native non-human antibodies which exhibit sufficiently high % sequence identity human germline sequences, as well as engineered, especially humanized, variants of such antibodies and also "fully human" antibodies.

**"Immunospecific", "specific for"** or to **"specifically bind"** - As used herein, an antibody is said to be "immunospecific", "specific for" or to "specifically bind" an antigen if it reacts at a detectable level with the antigen, preferably with an affinity constant, K_{A}, of greater than or equal to about 10⁶ M⁻¹, greater than or equal to about 10⁷ M⁻¹, or greater than or equal to 10⁸ M⁻¹, or greater than or equal to 1.5 10⁸ M^{-1,}, or greater than or equal to 10⁹ M⁻¹ or greater than or equal to 5 10⁹ M⁻¹. Affinity of an antibody for its cognate antigen is also commonly expressed as a dissociation constant K_{D}, and in certain embodiments, an antibody specifically binds to antigen if it binds with a K_{D} of less than or equal to 10⁻⁶ M, less than or equal to 10⁻⁷ M, or less than or equal to 1.5 10⁻⁸ M, or less than or equal to 10⁻⁸ M, or less than or equal to 5 10⁻⁹ M or less than or equal to 10⁻⁹ M. Affinities of antibodies can be readily determined using conventional techniques, for example, those described by Scatchard G et al. (The attractions of proteins for small molecules and ions. Ann NY Acad Sci 1949;51: 660-672). Binding properties of an antibody to antigens, cells or tissues thereof may generally be determined and assessed using immunodetection methods including, for example, ELISA, immunofluorescencebased assays, such as immuno-histochemistry (IHC) and/or fluorescence-activated cell sorting (FACS) or by surface plasmon resonance (SPR, BIAcore).

**"Inhibitor of GPVI signaling pathway"** - As used herein, this term includes any agent (e.g., chemical entity, protein, and the like) that, upon administration to a subject or to a cell induces an inhibition or down-regulation of a biological activity associated with activation of the GPVI signaling pathway, including any of the downstream biological effects otherwise resulting from the binding of the GPVI to its natural ligand.

**"Isolated nucleic acid"** - As used herein, an "isolated nucleic acid" is a nucleic acid that is substantially separated from other genome DNA sequences as well as proteins or complexes such as ribosomes and polymerases, which naturally accompany a native sequence. The term embraces a nucleic acid sequence that has been removed from its naturally occurring environment, and includes recombinant or cloned DNA isolates and chemically synthesized analogues or analogues biologically synthesized by heterologous systems. A substantially pure nucleic acid includes isolated forms of the nucleic acid. Of course, this refers to the nucleic acid as originally isolated and does not exclude genes or sequences later added to the isolated nucleic acid by the hand of man.

The term **"polypeptide"** is used in its conventional meaning, *i.e.,* as a sequence of less than 100 amino acids. A polypeptide usually refers to a monomeric entity. The term **"protein"** refers to a sequence of more than 100 amino acids and/or to a multimeric entity. The proteins of the invention are not limited to a specific length of the product. This term does not refer to or exclude post-expression modifications of the protein, for example, glycosylation, acetylation, phosphorylation and the like, as well as other modifications known in the art, both naturally occurring and non-naturally occurring. A protein may be an entire protein, or a subsequence thereof. Particular proteins of interest in the context of this invention are amino acid subsequences comprising CDRs and being capable of binding an antigen.

**"Identity"** or **"identical"** - As used herein, the term "identity" or "identical", when used in a relationship between the sequences of two or more amino acid sequences, refers to the degree of sequence relatedness between amino acid sequences, as determined by the number of matches between strings of two or more amino acid residues. "Identity" measures the percent of identical matches between the smaller of two or more sequences with gap alignments (if any) addressed by a particular mathematical model or computer program (*i.e.,* "algorithms"). Identity of related amino acid sequences can be readily calculated by known methods. Such methods include, but are not limited to, those described in Arthur M. Lesk, Computational Molecular Biology: Sources and Methods for Sequence Analysis (New-York: Oxford University Press, 1988); Douglas W. Smith, Biocomputing: Informatics and Genome Projects (New-York: Academic Press, 1993); Hugh G. Griffin and Annette M. Griffin, Computer Analysis of Sequence Data, Part 1 (New Jersey: Humana Press, 1994); Gunnar von Heinj e, Sequence Analysis in Molecular Biology: Treasure Trove or Trivial Pursuit (Academic Press, 1987); Michael Gribskov and John Devereux, Sequence Analysis Primer (New York: M. Stockton Press, 1991); and Carillo et al., 1988. SIAM J. Appl. Math. 48(5):1073-1082. Preferred methods for determining identity are designed to give the largest match between the sequences tested. Methods of determining identity are described in publicly available computer programs. Preferred computer program methods for determining identity between two sequences include the GCG program package, including GAP (Devereux et al., 1984. Nucl. Acid. Res. 12(1 Pt 1):387-395; Genetics Computer Group, University of Wisconsin Biotechnology Center, Madison, WI), BLASTP, BLASTN, TBLASTN and FASTA (Altschul et al., 1990. J. Mol. Biol. 215(3):403-410). The BLASTX program is publicly available from the National Center for Biotechnology Information (NCBI) and other sources (BLAST Manual, Altschul et al. NCB/NLM/NIH Bethesda, Md. 20894; Altschul et al., 1990. J. Mol. Biol. 215(3):403-410). The well-known Smith Waterman algorithm may also be used to determine identity.

**"Modified antibody"** - As used herein, the term "modified antibody" includes synthetic forms of antibodies which are altered such that they are not naturally occurring, *e.g.,* antibodies that comprise at least two heavy chain regions but not two complete heavy chains (such as, domain deleted antibodies or minibodies); multispecific forms of antibodies (*e.g.,* bispecific, trispecific, etc.) altered to bind to two or more different antigens or to different epitopes on a single antigen; heavy chain molecules joined to scFv molecules and the like. ScFv molecules are known in the art and are described, *e.g.,* in US patent 5,892,019. In addition, the term "modified antibody" includes multivalent forms of antibodies (*e.g.,* trivalent, tetravalent, etc., antibodies that bind to three or more copies of the same antigen).

**"Mammal"** - As used herein, the term "mammal" refers to any mammal, including humans, domestic and farm animals, and zoo, sports, or pet animals, such as dogs, cats, cattle, horses, sheep, pigs, goats, rabbits, etc. Preferably, the mammal is a primate, more preferably a human.

**"Monoclonal antibody"** - As used herein, the term "monoclonal antibody" refers to an antibody obtained from a population of substantially homogeneous antibodies, *i.e.,* the individual antibodies comprised in the population are identical except for possible naturally occurring mutations that may be present in minor amounts. Monoclonal antibodies are highly specific, being directed against a single antigenic site. Furthermore, in contrast to polyclonal antibody preparations that include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology first described by Kohler et al., Nature, 256:495 (1975), or may be made using recombinant DNA methods in bacterial, eukaryotic animal or plant cells (see, *e.g.,* U.S. Pat. No 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

**"Native sequence"** - As used herein, the term "native sequence" nucleotide refers to a polynucleotide that has the same nucleotide sequence as a polynucleotide derived from nature. Accordingly, a "native sequence" protein is one that has the same amino acid sequence as a protein (*e.g.,* antibody) derived from nature (*e.g.,* from any species). Such native sequence polynucleotides and proteins can be isolated from nature or can be produced by recombinant or synthetic means. A polynucleotide "variant", as the term is used herein, is a polynucleotide that typically differs from a polynucleotide specifically disclosed herein in one or more substitutions, deletions, additions and/or insertions. Such variants may be naturally occurring or may be synthetically generated, for example, by modifying one or more of the polynucleotide sequences as described herein and evaluating one or more biological activities of the encoded proteins as described herein and/or using any of a number of techniques well known in the art. A protein "variant", as the term is used herein, is a protein that typically differs from a protein specifically disclosed herein in one or more substitutions, deletions, additions and/or insertions. Such variants may be naturally occurring or may be synthetically generated, for example, by modifying one or more of the above protein sequences and evaluating one or more biological activities of the protein as described herein and/or using any of a number of techniques well known in the art. Modifications may be made in the structure of the polynucleotides and proteins of the present invention and still obtain a functional molecule that encodes a variant or derivative protein with desirable characteristics. When it is desired to alter the amino acid sequence of a protein to create an equivalent, or even an improved, variant or region of a protein as described herein, one skilled in the art will typically change one or more of the codons of the encoding DNA sequence. For example, certain amino acids may be substituted for other amino acids in a protein structure without appreciable loss of its ability to bind other proteins (*e.g.,* antigens) or cells. Since it is the binding capacity and nature of a protein that defines that protein's biological functional activity, certain amino acid sequence substitutions can be made in a protein sequence, and of course, its underlying DNA coding sequence, and nevertheless obtain a protein with similar properties. It is thus contemplated that various changes may be made in the amino acid sequences of the disclosed compositions, or corresponding DNA sequences that encode said proteins without appreciable loss of their biological utility or activity. In many instances, a protein variant will contain one or more conservative substitutions. In a preferred embodiment, variant proteins differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the protein.

**"Pharmaceutically acceptable excipient"** - As used herein, the term "pharmaceutically acceptable excipient" includes any and all solvents, dispersion media, coatings, antibacterial and antifungal agents, isotonic and absorption delaying agents and the like. Said excipient does not produce an adverse, allergic or other untoward reaction when administered to an animal, preferably a human. For human administration, preparations should meet sterility, pyrogenicity, and general safety and purity standards as required by regulatory offices, such as, for example, FDA Office or EMA.

**"Specificity"** - As used herein, the term "specificity" refers to the ability to specifically bind (*e.g.,* immunoreact with) a given target, *e.g.,* GPVI. A protein may be monospecific and contain one or more binding sites which specifically bind a target, or a protein may be multispecific and contain two or more binding sites which specifically bind the same or different targets. In an embodiment, an antibody as described herein is specific for more than one target. For example, in an embodiment, a multispecific binding molecule binds to GPVI and a second molecule.

**"Single-chain Fv"** also abbreviated as "sFv" or "scFv" - As used herein, the terms "Single-chain Fv", "sFv" or "scFv" are antibody fragments that comprise the VH and VL antibody domains connected into a single amino acid chain. Preferably, the sFv amino acid sequence further comprises a peptidic linker between the VH and VL domains that enables the sFv to form the desired structure for antigen binding.

**"Subject"** - As used herein, the term "subject" refers to a mammal, preferably a human. In one embodiment, a subject may be a "patient", *i.e.,* a warm-blooded animal, more preferably a human, who/which is awaiting the receipt of, or is receiving medical care or was/is/will be the object of a medical procedure, or is monitored for the development of a disease.

**"Synthetic"** - As used herein, the term "synthetic" with respect to proteins includes proteins which comprise an amino acid sequence that is not naturally occurring. For example, non-naturally occurring proteins are modified forms of naturally occurring proteins (*e.g.,* comprising a mutation such as an addition, substitution or deletion) or proteins which comprise a first amino acid sequence (which may or may not be naturally occurring) that is linked in a linear sequence of amino acids to a second amino acid sequence (which may or may not be naturally occurring) to which it is not naturally linked in nature.

**"Therapeutically effective amount"** means level or amount of agent that is aimed at, without causing significant negative or adverse side effects to the target, (1) delaying or preventing the onset of GPVI-related condition; (2) slowing down or stopping the progression, aggravation, or deterioration of one or more symptoms of the GPVI-related condition; (3) bringing about ameliorations of the symptoms of the GPVI-related condition; (4) reducing the severity or incidence of the GPVI-related condition; or (5) curing the GPVI-related condition. A therapeutically effective amount may be administered prior to the onset of the GPVI-related condition, for a prophylactic or preventive action. Alternatively or additionally, the therapeutically effective amount may be administered after initiation of the GPVI-related condition, for a therapeutic action.

**"Treating"** or **"treatment"** or **"alleviation"** - As used herein, the terms "treating" or "treatment" or "alleviation" refers to both therapeutic treatment and prophylactic or preventative measures; wherein the object is to prevent or slow down (lessen) the targeted pathologic condition or disorder or to prevent or limit the consequences of the targeted pathologic condition (in particular disability). Those in need of treatment include those already with the disorder as well as those prone to have the disorder or those in whom the disorder is to be prevented. In some embodiments, a subject or mammal is successfully "treated" for the targeted pathologic condition or disorder if, after receiving a therapeutic amount of a protein according to the present invention, the subject or mammal shows observable and/or measurable improvement in one or more of the following: reduction in the number of pathogenic cells; reduction in the percent of total cells that are pathogenic; and/or relief to some extent, of one or more of the symptoms associated with the specific disease or condition; reduced morbidity and mortality, and/or improvement in quality of life issues. The above parameters for assessing successful treatment and improvement in the disease are readily measurable by routine procedures familiar to a physician.

**"Variable region"** or **"variable domain"** - As used herein, the term "variable" refers to the fact that certain regions of the variable domains VH and VL differ extensively in sequence among antibodies and are used in the binding and specificity of each particular antibody for its target antigen. However, the variability is not evenly distributed throughout the variable domains of antibodies. It is concentrated in three segments called "hypervariable loops" in each of the VL domain and the VH domain which form part of the antigen binding site.

**"Valency"** - As used herein, the term "valency" refers to the number of potential target binding sites in a protein. Each target binding site specifically binds one target molecule or specific site on a target molecule. When a protein comprises more than one target binding site, each target binding site may specifically bind the same or different molecules (e.g., may bind to different ligands or different antigens, or different epitopes on the same antigen). The subject binding molecules preferably have at least one binding site specific for a human GPVI molecule. Preferably, the proteins provided herein are monovalent.

### DETAILED DESCRIPTION

An object of the present invention is an inhibitor of the Glycoprotein VI (GPVI) signaling pathway for treating or for use in treating a GPVI-related condition in a subject in need thereof, wherein said subject presents at least one of the following conditions:
i) said subject has a large ischemic core volume, and/or
ii) said subject has a good cerebral collateral circulation.

As used herein, the term "inhibit" means that the inhibitor for use in the present invention is capable of blocking, reducing, preventing or neutralizing GPVI interaction with its ligands, GPVI signaling and/or the activation of molecules from the GPVI signaling pathway.

Examples of ligands of GPVI include, but are not limited to, collagen, fibrin, fibronectin, vitronectin and laminins.

In one embodiment, the inhibitor of GPVI signaling pathway acts by inhibiting the expression of GPVI and/or its ligand.

In one embodiment, the term "inhibit" refers to a decreased expression level as compared to a reference expression level, such as, for example, a level inferior or equal to 90%, 80%, 70%, 60%, 50%, 40%, 30%, 20%, 10%, or less of the reference expression level. In one embodiment, the inhibition of expression of GPVI and/or its ligand is measured after contacting a cell with a compound tested for its impact on expression, and the reference expression level correspond to an expression level measured in a cell not contacted with said compound.

Methods to determine the level of gene expression are well-known to the skilled artisan, and include, without limitation, determining the transcriptome, and/or the proteome.

*In vitro* methods for assessing the transcription level of a gene are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like.

*In vitro* methods for assessing the translation level of a gene are well-known in the art. Examples of such methods include, but are not limited to, immunohistochemistry, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

Examples of inhibitors of the gene expression of GPVI and/or its ligand include, without limitation, small inhibitory RNAs (siRNA), micro RNAs (miRNA) short hairpin RNAs (shRNA), oligonucleotide antisense (including, without limitation, antisense RNA or DNA molecules), ribozymes, aptamers, morpholinos, and engineered nucleases.

In one embodiment, the inhibitor of GPVI signaling pathway inhibits the binding of GPVI to a ligand thereof (such as, for example, collagen, fibrin or any other GPVI ligand capable of inducing downstream signaling and platelet activation).

In one embodiment, the inhibitor of GPVI signaling pathway binds a ligand of GPVI, preferably human GPVI (hGPVI).

In one embodiment, the inhibitor of GPVI signaling pathway acts by occupying the GPVI binding site or a portion thereof of the ligand, thereby making the ligand inaccessible to GPVI, so that its normal biological activity is prevented or reduced.

In one embodiment the inhibitor of GPVI signaling pathway binds a ligand of GPVI selected from the group comprising collagen, fibrin, fibronectin, vitronectin and laminins.

In one embodiment, the inhibitor of GPVI signaling pathway binds Glycoprotein VI (GPVI), preferably hGPVI.

In one embodiment, the inhibitor of GPVI signaling pathway may be a competitive inhibitor, a non-competitive inhibitor, or an uncompetitive inhibitor.

In one embodiment, the inhibitor of GPVI signaling pathway is a competitive inhibitor and acts by occupying the ligand binding site or a portion thereof of the receptor (e.g. hGPVI), thereby making the receptor inaccessible to its natural ligand, so that its normal biological activity is prevented or reduced.

In one embodiment, the inhibitor of GPVI signaling pathway is a non-competitive inhibitor that reduces the activity of a receptor (e.g., hGPVI) and that binds equally well to the receptor whether or not it has already bound its ligand.

In one embodiment, the inhibitor of GPVI signaling pathway is an uncompetitive inhibitor that binds only to the complex formed between the receptor (e.g., hGPVI) and its ligand, acting typically in reactions with two or more substrates or products.

Examples of inhibitors of the GPVI signaling pathway include, without limitation, proteins, peptides, polypeptides and small organic molecules.

In one embodiment the inhibitor of the GPVI signaling pathway is an isolated protein, preferably an isolated humanized protein, binding to hGPVI.

Thus, an object of the present invention is an isolated humanized protein binding to hGPVI for treating or for use in treating a GPVI-related condition in a subject in need thereof, wherein said subject presents at least one of the following conditions:
i) said subject has a large ischemic core volume, and/or
ii) said subject has a good cerebral collateral circulation.

In one embodiment, the protein binding to hGPVI binds to the extracellular domain of GPVI.

In one embodiment, the protein binding to hGPVI binds to the Ig-like C2-type domain 2 (D2) of human GPVI.

In one embodiment, the protein binding to hGPVI binds to a conformational epitope.

In one embodiment, the protein binding to hGPVI has a K_{D} for binding to human GPVI, preferably soluble human GPVI, less than or equal to 15 nM, preferably less than or equal to 10 nM and more preferably less than or equal to 5 nM.

In one embodiment, the protein binding to hGPVI has a k_{off} for binding to human GPVI of less than or equal to about 8.10⁻⁴ sec⁻¹, preferably less than or equal to about 6.10⁻⁴ sec⁻¹, and more preferably less than or equal to about 4.10⁻⁴ sec⁻¹.

In one embodiment, the protein binding to hGPVI has a kₒₙ for binding to human GPVI of at least about 5.10⁴ M⁻¹sec⁻¹, preferably at least about 5.5.10⁴ M⁻¹sec⁻¹, and more preferably at least about 5.9.10⁴ M⁻¹sec⁻¹ and more preferably at least about 6.10⁻⁴ sec⁻¹.

In one embodiment, the K_{D} may be determined by Surface plasmon resonance (SPR, BIAcore), using immobilized soluble GPVI at a dose ranging from about 700 to about 1600 resonance units (RU) (corresponding to about 8.5 to about 11.3 fmol/mm²), preferably from about 850 to about 1200 RU, more preferably from about 950 to about 1075 RU and/or using PBS pH 7.4 as running buffer, and/or using BIAevaluation version 3.0 software for analyzing data. In one embodiment, soluble GPVI corresponds to the extracellular domain of GPVI fused at its C-terminus via a linker (such as, for example, via a Gly-Gly-Arg linker) to a hFc sequence. This soluble GPVI may be referred to as soluble GPVI-Fc.

Methods for determining the affinity of a protein for a ligand are well known in the art. An example of a method for determining the affinity of a protein for soluble GPVI is shown below:

Binding of the protein to soluble human GPVI is analyzed with surface plasmon resonance using a BIAcore 2000 system (Uppsala, Sweden).

Soluble GPVI-Fc is immobilized at a dose ranging from about 700 to about 1600 RU (corresponding to about 8.5 to about 11.3 fmol/mm²), preferably from about 850 to about 1200 RU, more preferably from about 950 to about 1075 RU, and even more preferably from about 960 to about 1071 RU onto a Carboxy-Methyl Dextran CM5 sensor chip using the amine coupling method (Wizard procedure). The protein is then passed over the immobilized GPVI-Fc in PBS pH 7.4 (10 mM phosphate, 138 mM NaCl, 2.7 mM KCl, pH 7.42 at 25.4°C) at a flow rate of 20 µL/min at 25°C. Kinetic constants (kₒₙ, k_{off}) and affinity are determined using BIAevaluation version 3.0 software, by fitting data to binding model. PBS pH 7.4 is the running buffer.

In one embodiment, the protein binding to hGPVI is an antibody molecule or a fragment thereof selected from the group comprising or consisting of a whole antibody, a single chain variable fragment (ScFv), a single chain antibody, a dimeric single chain antibody, a Fv fragment, a Fab fragment, a F(ab)'₂ fragment, a defucosylated antibody, a bi-specific antibody, a diabody, a triabody and a tetrabody.

In another embodiment, the protein binding to hGPVI is an antibody fragment selected from the group comprising or consisting of a unibody, a domain antibody, and a nanobody.

In one embodiment, the protein binding to hGPVI is an antibody molecule or an antibody fragment selected from the group comprising or consisting of a whole antibody, a single chain variable fragment (ScFv), a Fv fragment, a Fab fragment, a unibody, a domain antibody, and a nanobody, preferably selected from the group comprising or consisting of a whole antibody, a single chain variable fragment (ScFv), a Fv fragment, a Fab fragment, and a unibody.

In another embodiment, the protein binding to hGPVI is an antibody mimetic selected from the group comprising or consisting of an affibody, an affilin, an affitin, an adnectin, an atrimer, an evasin, a DARPin, an anticalin, an avimer, a fynomer, a versabody and a duocalin.

A domain antibody is well known in the art and refers to the smallest functional binding units of antibodies, corresponding to the variable regions of either the heavy or light chains of antibodies.

A nanobody is well known in the art and refers to an antibody-derived therapeutic protein that contains the unique structural and functional properties of naturally-occurring heavy chain antibodies. These heavy chain antibodies may contain a single variable domain (VHH) and two constant domains (CH2 and CH3).

A unibody is well known in the art and refers to an antibody fragment lacking the hinge region of IgG4 antibodies. The deletion of the hinge region results in a molecule that is essentially half the size of traditional IgG4 antibodies and has a univalent binding region rather than the bivalent biding region of IgG4 antibodies.

An affibody is well known in the art and refers to affinity proteins based on a 58 amino acid residue protein domain, derived from one of the IgG binding domain of staphylococcal protein A.

DARPins (Designed Ankyrin Repeat Proteins) are well known in the art and refer to an antibody mimetic DRP (designed repeat protein) technology developed to exploit the binding abilities of non-antibody proteins.

Anticalins are well known in the art and refer to another antibody mimetic technology, wherein the binding specificity is derived from lipocalins. Anticalins may also be formatted as dual targeting protein, called Duocalins.

Avimers are well known in the art and refer to another antibody mimetic technology.

Versabodies are well known in the art and refer to another antibody mimetic technology. They are small proteins of 3-5 kDa with >15% cysteines, which form a high disulfide density scaffold, replacing the hydrophobic core the typical proteins have.

In one embodiment, the protein binding to hGPVI is a monovalent antibody or fragment thereof.

In one embodiment, the protein binding to hGPVI is a monoclonal antibody or a fragment thereof.

In another embodiment, the protein binding to hGPVI is a polyclonal antibody or a fragment thereof.

An object of the present invention is an anti-hGPVI antibody or a fragment thereof for treating or for use in treating a GPVI-related condition in a subject in need thereof, wherein said subject presents at least one of the following conditions:
i) said subject has a large ischemic core volume, and/or
ii) said subject has a good cerebral collateral circulation.

In one embodiment, the variable region of the heavy chain of the anti-hGPVI antibody or fragment thereof comprises at least one of the followings CDRs:
VH-CDR1: GYTFTSYNMH (SEQ ID NO: 1);
VH-CDR2: GIYPGNGDTSYNQKFQG (SEQ ID NO: 2); and
VH-CDR3: GTVVGDWYFDV (SEQ ID NO: 3).

In one embodiment, the variable region of the light chain of the anti-hGPVI antibody or fragment thereof comprises at least one of the followings CDRs:
VL-CDR1: RSSQSLENSNGNTYLN (SEQ ID NO: 4);
VL-CDR2: RVSNRFS (SEQ ID NO: 5); and
VL-CDR3: LQLTHVPWT (SEQ ID NO: 6).

As used herein, CDR numbering and definition are according to the Kabat/Chothia definition.

In one embodiment, the anti-hGPVI antibody or fragment thereof comprises:
- in the heavy chain, at least one of the following CDR: GYTFTSYNMH (SEQ ID NO: 1), GIYPGNGDTSYNQKFQG (SEQ ID NO: 2) and GTVVGDWYFDV (SEQ ID NO: 3); and/or
- in the light chain, at least one of the following CDR: RSSQSLENSNGNTYLN (SEQ ID NO: 4), RVSNRFS (SEQ ID NO: 5), and LQLTHVPWT (SEQ ID NO: 6).

In another embodiment of the invention, the anti-hGPVI antibody or fragment thereof comprises in its heavy chain the 3 CDRs SEQ ID NO: 1, SEQ ID NO: 2 and SEQ ID NO: 3.

In another embodiment of the invention, the anti-hGPVI antibody or fragment thereof comprises in its light chain the 3 CDRs SEQ ID NO: 4, SEQ ID NO: 5 and SEQ ID NO: 6.

In another embodiment of the invention, the anti-hGPVI antibody or fragment thereof comprises in its heavy chain the 3 CDRs SEQ ID NO: 1, SEQ ID NO: 2 and SEQ ID NO: 3, and in its light chain the 3 CDRs SEQ ID NO: 4, SEQ ID NO: 5 and SEQ ID NO: 6.

In another embodiment of the invention, the anti-hGPVI antibody or fragment thereof comprises in its heavy chain the 3 CDRs SEQ ID NO: 1, SEQ ID NO: 2 and SEQ ID NO: 3, and in its light chain the 3 CDRs SEQ ID NO: 4, SEQ ID NO: 5 and SEQ ID NO: 6, optionally wherein one, two, three or more of the amino acids in any of said sequences may be substituted by a different amino acid.

According to the invention, any of the CDRs 1, 2 and 3 of the heavy and light chains may be characterized as having an amino acid sequence that shares at least 60%, 70%, 75%, 80%, 90%, 95%, 96%, 97%, 98%, 99% of identity with the particular CDR or sets of CDRs listed in the corresponding SEQ ID NO.

In another embodiment of the invention, the anti-hGPVI antibody or fragment thereof is an antibody or fragment thereof having:
(i) the heavy chain CDR 1, 2 and 3 (VH-CDR1, VH-CDR2, VH-CDR3) amino acid sequences as shown in SEQ ID NO: 1, 2 and 3 respectively; and
(ii) the light chain CDR 1, 2 and 3 (VL-CDR1, VL-CDR2, VL-CDR3) amino acid sequences as shown in SEQ ID NO: 4, 5 and 6 respectively;
optionally wherein one, two, three or more of the amino acids in any of said sequences may be substituted by a different amino acid.

In one embodiment, the anti-hGPVI antibody or fragment thereof comprises a heavy chain variable region comprising or consisting of the sequence SEQ ID NO: 7.

SEQ ID NO: 7
QVQLVQSGAEVKKPGASVKVSCKASGYTFTSYNMHWVRQAPGQGLEWMGGI
YPGNGDTSYNQKFQGRVTMTRDTSTSTVYMELSSLRSEDTAVYYCARGTVVG
DWYFDVWGQGTLVTVSS.

In one embodiment, the anti-hGPVI antibody or fragment thereof comprises a light chain variable region comprising or consisting of the sequence SEQ ID NO: 8.

SEQ ID NO: 8
DIQMTQSPSSLSASVGDRVTITCRSSQSLENSNGNTYLNWYQQKPGKAPKLLIY
RVSNRFSGVPSRFSGSGSGTDFTFTISSLQPEDIATYYCLQLTHVPWTFGQGTKV
EITR.

In one embodiment, the anti-hGPVI antibody or fragment thereof comprises a light chain variable region comprising or consisting of the sequence SEQ ID NO: 9.

SEQ ID NO: 9
DIQMTQSPSSLSASVGDRVTITCSASQSLENSNGNTYLNWYQQKPGKAPKLLIY
RVSNRFSGVPSRFSGSGSGTDFTLTISSLQPEDFATYYCLQLTHVPWTFGQGTKV
EIKR.

In another embodiment of the invention, the anti-hGPVI antibody (ACT017 antibody) or fragment thereof comprises a heavy chain variable region comprising or consisting of the sequence SEQ ID NO: 7 and the light chain variable region comprising or consisting of the sequence SEQ ID NO: 8.

In another embodiment of the invention, the anti-hGPVI antibody (ACT006 antibody) or fragment thereof comprises the heavy chain variable region comprising or consisting of the sequence SEQ ID NO: 7 and the light chain variable region comprising or consisting of the sequence SEQ ID NO: 9.

According to the invention, one, two, three or more of the amino acids of the heavy chain or light chain variable regions as described hereinabove may be substituted by a different amino acid.

In another embodiment, an antibody (or a fragment thereof) for use in the present invention comprises heavy and light chain variable regions comprising amino acid sequences that are homologous to the amino acid sequences of the ACT017 antibody described herein, and wherein the antibodies (or fragments thereof) retain the desired functional properties of the protein described in the present invention.

In another embodiment, an antibody (or a fragment thereof) for use in the present invention comprises heavy and light chain variable regions comprising amino acid sequences that are homologous to the amino acid sequences of the ACT006 antibody described herein, and wherein the antibodies (or fragments thereof) retain the desired functional properties of the protein described in the present invention.

According to the invention, the heavy chain variable region encompasses sequences that have 60%, 70%, 75%, 80%, 90%, 95%, 96%, 97%, 98%, 99% or more of identity with SEQ ID NO: 7.

According to the invention, the light chain variable region encompasses sequences that have 60%, 70%, 75%, 80%, 90%, 95%, 96%, 97%, 98%, 99% or more of identity with SEQ ID NO: 8 or SEQ ID NO: 9.

In any of the antibodies or fragments thereof for use in the present invention (e.g. ACT017 or ACT006), the specified variable region and CDR sequences may comprise conservative sequence modifications. Conservative sequence modifications refer to amino acid modifications that do not significantly affect or alter the binding characteristics of the antibody (or fragment thereof) containing the amino acid sequence. Such conservative modifications include amino acid substitutions, additions and deletions. Modifications can be introduced into an antibody (or fragment thereof) for use in the present invention by standard techniques known in the art, such as site-directed mutagenesis and PCR-mediated mutagenesis.

Thus, one or more amino acid residues within the CDR regions of an antibody (or fragment thereof) for use in the present invention can be replaced with other amino acid residues from the same side chain family and the altered antibody (or fragment thereof) can be tested for retained function (i.e., the properties set forth herein) using the assays described herein.

In one embodiment, the antibody (or fragment thereof) for use in the present invention binds essentially the same epitope as ACT017 or ACT006 antibodies. In the present invention, an antibody (or fragment thereof) that binds essentially the same epitope as ACT017 or ACT006 antibodies will be referred as an ACT017-like or ACT006-like antibody, respectively.

In one embodiment, the antibody (or fragment thereof) for use in the present invention competes for binding to hGPVI with an antibody as described hereinabove, in particular with an antibody selected from ACT017 and ACT006.

In some embodiments of this invention, anti-hGPVI antibodies or fragments thereof comprising VH and VL domains, or CDRs thereof may comprise CH1 domains and/or CL domains, the amino acid sequence of which is fully or substantially human. Where the GPVI binding protein is an antibody (or fragment thereof) intended for human therapeutic use, it is typical for the entire constant region of the antibody, or at least a part thereof, to have a fully or substantially human amino acid sequence. Therefore, one or more or any combination of the CH1 domain, hinge region, CH2 domain, CH3 domain and CL domain (and CH4 domain if present) may be fully or substantially human with respect to its amino acid sequence. Advantageously, the CH1 domain, hinge region, CH2 domain, CH3 domain and CL domain (and CH4 domain if present) may all have a fully or substantially human amino acid sequence. In the context of the constant region of a humanized or chimeric antibody, or an antibody fragment, the term "substantially human" refers to an amino acid sequence identity of at least 70%, or at least 80%, or at least 90%, or at least 95%, or at least 97%, or at least 99% with a human constant region. The term "human amino acid sequence" in this context refers to an amino acid sequence which is encoded by a human immunoglobulin gene, which includes germline, rearranged and somatically mutated genes. The invention also contemplates the use of proteins comprising constant domains of "human" sequence which have been altered, by one or more amino acid additions, deletions or substitutions with respect to the human sequence, excepting those embodiments where the presence of a "fully human" hinge region is expressly required. The presence of a "fully human" hinge region in the anti-hGPVI antibodies for use in the present invention may be beneficial both to minimize immunogenicity and to optimize stability of the antibody. It is considered that one or more amino acid substitutions, insertions or deletions may be made within the constant region of the heavy and/or the light chain, particularly within the Fc region. Amino acid substitutions may result in replacement of the substituted amino acid with a different naturally occurring amino acid, or with a non-natural or modified amino acid. Other structural modifications are also permitted, such as for example changes in glycosylation pattern *(e.g.,* by addition or deletion of N- or O-linked glycosylation sites). Depending on the intended use of the antibody, it may be desirable to modify the antibody with respect to its binding properties to Fc receptors, for example to modulate effector function. For example, cysteine residue(s) may be introduced in the Fc region, thereby allowing interchain disulfide bond formation in this region. The homodimeric antibody thus generated may have improved effector function.

Thus, in one embodiment, the anti-hGPVI antibody or fragment thereof is humanized or chimeric.

In one embodiment, the heavy chain variable region of sequence SEQ ID NO: 7 is encoded by SEQ ID NO: 10:

SEQ ID NO: 10
CAGGTTCAGCTGGTTCAGTCAGGGGCTGAGGTGAAGAAGCCTGGAGCCTCA
GTGAAGGTGTCCTGCAAGGCTTCTGGCTACACATTTACCAGTTACAATATGC
ACTGGGTAAGACAGGCTCCTGGACAGGGCCTGGAATGGATGGGAGGTATTT
ATCCAGGAAATGGTGATACTTCCTACAATCAGAAGTTCCAGGGCCGAGTTA
CTATGACTCGGGACACTTCCACCTCTACAGTGTACATGGAGCTCAGCAGCCT
GAGATCTGAGGACACCGCGGTCTATTACTGTGCAAGAGGCACCGTGGTCGG
CGACTGGTACTTCGATGTGTGGGGCCAAGGCACCCTGGTCACCGTGAGCAG
T.

In one embodiment, the light chain variable region of sequence SEQ ID NO: 8 is encoded by SEQ ID NO: 11:

SEQ ID NO: 11
GACATCCAGATGACCCAGAGCCCAAGCAGCCTGAGCGCCAGCGTGGGTGAC
AGAGTGACCATCACCTGTAGAAGTAGTCAGAGCCTTGAGAACAGCAACGGA
AACACCTACCTGAATTGGTACCAGCAGAAGCCAGGTAAGGCTCCAAAGCTG
CTGATCTACAGAGTTTCCAACCGATTCTCTGGTGTGCCAAGCAGATTCAGCG
GTAGCGGTAGCGGTACCGACTTCACCTTCACCATCAGCAGCCTCCAGCCAG
AGGACATCGCCACCTACTACTGCCTCCAGCTGACTCATGTCCCATGGACCTT
CGGTCAGGGCACCAAGGTGGAGATCACCCGG.

In one embodiment, the light chain variable region of sequence SEQ ID NO: 9 is encoded by SEQ ID NO: 12.

SEQ ID NO: 12
GACATCCAGATGACCCAGAGCCCAAGCAGCCTGAGCGCCAGCGTGGGTGAC
AGAGTGACCATCACCTGTAGTGCCAGTCAGAGCCTTGAGAACAGCAACGGA
AACACCTACCTGAATTGGTACCAGCAGAAGCCAGGTAAGGCTCCAAAGCTG
CTGATCTACAGAGTTTCCAACCGATTCTCTGGTGTGCCAAGCAGATTCAGCG
GTAGCGGTAGCGGTACCGACTTCACCCTCACCATCAGCAGCCTCCAGCCAG
AGGACTTCGCCACCTACTACTGCCTCCAGCTGACTCATGTCCCATGGACCTT
CGGTCAGGGCACCAAGGTGGAGATCAAACGC.

In one embodiment, the nucleic sequences encoding the anti-hGPVI antibody or fragment thereof are comprised in an expression vector. In one embodiment, the expression vector comprises at least one of SEQ ID NO: 10, SEQ ID NO: 11 and SEQ ID NO: 12 or any sequence having a nucleic acid sequence that shares at least 60%, 70%, 75%, 80%, 90%, 95%, 96%, 97%, 98%, 99% of identity with said SEQ ID NO: 10-12.

In one embodiment, the expression vector comprises SEQ ID NO: 10 and SEQ ID NO: 11 or any sequence having a nucleic acid sequence that shares at least 60%, 70%, 75%, 80%, 90%, 95%, 96%, 97%, 98%, 99% of identity with said SEQ ID NO: 10-11.

In one embodiment, the expression vector comprises SEQ ID NO: 10 and SEQ ID NO: 12 or any sequence having a nucleic acid sequence that shares at least 60%, 70%, 75%, 80%, 90%, 95%, 96%, 97%, 98%, 99% of identity with said SEQ ID NO: 10, 12.

In one embodiment, the vector comprises the sequence SEQ ID NO: 10 and a sequence encoding a constant region of a heavy chain. A non-limiting example of a sequence encoding a constant region of a heavy chain is SEQ ID NO: 14.

SEQ ID NO: 14
GCCTCCACCAAGGGTCCCTCAGTCTTCCCACTGGCACCCTCCTCCAAGAGCA
CCTCTGGTGGCACAGCTGCCCTGGGCTGCCTGGTCAAGGACTACTTCCCAGA
ACCAGTGACTGTGTCATGGAACTCAGGCGCCCTGACCAGCGGCGTGCACAC
CTTCCCTGCTGTCTTGCAGTCCTCAGGACTCTACTCCCTCAGCAGCGTGGTG
ACCGTGCCCTCCAGCAGCTTGGGCACCCAGACCTACATCTGCAACGTGAAT
CACAAGCCCAGCAACACCAAGGTGGACAAGAAAGTCGAGCCTAAGTCATG
CGACAAGACTCAC.

In one embodiment, the vector comprises the sequence SEQ ID NO: 11 or SEQ ID NO: 12 and a sequence encoding a constant region of a light chain. A non-limiting example of a sequence encoding a constant region of a light chain is SEQ ID NO: 15.

SEQ ID NO: 15
ACTGTGGCTGCACCAAGTGTGTTCATCTTCCCACCTAGCGATGAGCAGTTGA
AATCTGGAACTGCCTCTGTCGTGTGCCTCCTGAACAACTTCTACCCACGGGA
GGCCAAGGTACAGTGGAAGGTGGATAACGCCCTCCAATCCGGTAACTCCCA
GGAGAGTGTCACAGAGCAAGATAGCAAGGACAGCACCTACAGCCTCAGCA
GCACCCTGACTCTGAGCAAAGCAGACTACGAGAAGCACAAGGTCTACGCCT
GCGAAGTCACCCATCAGGGCCTGAGTTCCCCTGTCACAAAGAGCTTCAACC
GGGGAGAGTGT.

In one embodiment, the vector comprises a sequence encoding a signal peptide. Non-limiting examples of signal peptides sequences include, but are not limited to, SEQ ID NO: 16 and SEQ ID NO: 17.
SEQ ID NO: 16 
ATGGATATGCGTGTACCAGCTCAACTACTTGGACTTCTATTGCTTTGGCTTC
GTGGTGCTAGATGT.
SEQ ID NO: 17

In one embodiment, the vector comprises SEQ ID NO: 10, and a sequence encoding a constant region of a heavy chain (such as, for example, SEQ ID NO: 14), and a signal peptide sequence. An example of such a vector is a vector comprising SEQ ID NO: 18. SEQ ID NO: 18 further comprises cloning sites.

SEQ ID NO: 18
GCGGCCGCCACCATGGACTGGACTTGGAGAATCCTATTCTTGGTTGCTGCAG
CTACAGGTGCTCATTCACAGGTTCAGCTGGTTCAGTCAGGGGCTGAGGTGA
AGAAGCCTGGAGCCTCAGTGAAGGTGTCCTGCAAGGCTTCTGGCTACACAT
TTACCAGTTACAATATGCACTGGGTAAGACAGGCTCCTGGACAGGGCCTGG
AATGGATGGGAGGTATTTATCCAGGAAATGGTGATACTTCCTACAATCAGA
AGTTCCAGGGCCGAGTTACTATGACTCGGGACACTTCCACCTCTACAGTGTA
CATGGAGCTCAGCAGCCTGAGATCTGAGGACACCGCGGTCTATTACTGTGC
AAGAGGCACCGTGGTCGGCGACTGGTACTTCGATGTGTGGGGCCAAGGCAC
CCTGGTCACCGTGAGCAGTGCCTCCACCAAGGGTCCCTCAGTCTTCCCACTG
GCACCCTCCTCCAAGAGCACCTCTGGTGGCACAGCTGCCCTGGGCTGCCTGG
TCAAGGACTACTTCCCAGAACCAGTGACTGTGTCATGGAACTCAGGCGCCC
TGACCAGCGGCGTGCACACCTTCCCTGCTGTCTTGCAGTCCTCAGGACTCTA
CTCCCTCAGCAGCGTGGTGACCGTGCCCTCCAGCAGCTTGGGCACCCAGAC
CTACATCTGCAACGTGAATCACAAGCCCAGCAACACCAAGGTGGACAAGAA
AGTCGAGCCTAAGTCATGCGACAAGACTCACTGATGAGGATCC.

In one embodiment, the vector comprises SEQ ID NO: 11, and a sequence encoding a constant region of a light chain (such as, for example, SEQ ID NO: 15), and a signal peptide sequence. An example of such a vector is a vector comprising SEQ ID NO: 19. SEQ ID NO: 19 further comprises cloning sites.

SEQ ID NO: 19
GACGTCACCATGGATATGCGTGTACCAGCTCAACTACTTGGACTTCTATTGC
TTTGGCTTCGTGGTGCTAGATGTGACATCCAGATGACCCAGAGCCCAAGCA
GCCTGAGCGCCAGCGTGGGTGACAGAGTGACCATCACCTGTAGAAGTAGTC
AGAGCCTTGAGAACAGCAACGGAAACACCTACCTGAATTGGTACCAGCAGA
AGCCAGGTAAGGCTCCAAAGCTGCTGATCTACAGAGTTTCCAACCGATTCTC
TGGTGTGCCAAGCAGATTCAGCGGTAGCGGTAGCGGTACCGACTTCACCTT
CACCATCAGCAGCCTCCAGCCAGAGGACATCGCCACCTACTACTGCCTCCA
GCTGACTCATGTCCCATGGACCTTCGGTCAGGGCACCAAGGTGGAGATCAC
CCGGACTGTGGCTGCACCAAGTGTGTTCATCTTCCCACCTAGCGATGAGCAG
TTGAAATCTGGAACTGCCTCTGTCGTGTGCCTCCTGAACAACTTCTACCCAC
GGGAGGCCAAGGTACAGTGGAAGGTGGATAACGCCCTCCAATCCGGTAACT
CCCAGGAGAGTGTCACAGAGCAAGATAGCAAGGACAGCACCTACAGCCTC
AGCAGCACCCTGACTCTGAGCAAAGCAGACTACGAGAAGCACAAGGTCTAC
GCCTGCGAAGTCACCCATCAGGGCCTGAGTTCCCCTGTCACAAAGAGCTTC
AACCGGGGAGAGTGTTGATGATATC.

In one embodiment, the vector comprises SEQ ID NO: 12, and a sequence encoding a constant region of a light chain (such as, for example, SEQ ID NO: 15), and a signal peptide sequence. An example of such a vector is a vector comprising SEQ ID NO: 20. SEQ ID NO: 20 further comprises cloning sites.

SEQ ID NO: 20
GACGTCACCATGGATATGCGTGTACCAGCTCAACTACTTGGACTTCTATTGC
TTTGGCTTCGTGGTGCTAGATGTGACATCCAGATGACCCAGAGCCCAAGCA
GCCTGAGCGCCAGCGTGGGTGACAGAGTGACCATCACCTGTAGTGCCAGTC
AGAGCCTTGAGAACAGCAACGGAAACACCTACCTGAATTGGTACCAGCAGA
AGCCAGGTAAGGCTCCAAAGCTGCTGATCTACAGAGTTTCCAACCGATTCTC
TGGTGTGCCAAGCAGATTCAGCGGTAGCGGTAGCGGTACCGACTTCACCCT
CACCATCAGCAGCCTCCAGCCAGAGGACTTCGCCACCTACTACTGCCTCCAG

CTGACTCATGTCCCATGGACCTTCGGTCAGGGCACCAAGGTGGAGATCAAA
CGCACTGTGGCTGCACCAAGTGTGTTCATCTTCCCACCTAGCGATGAGCAGT
TGAAATCTGGAACTGCCTCTGTCGTGTGCCTCCTGAACAACTTCTACCCACG
GGAGGCCAAGGTACAGTGGAAGGTGGATAACGCCCTCCAATCCGGTAACTC
CCAGGAGAGTGTCACAGAGCAAGATAGCAAGGACAGCACCTACAGCCTCA
GCAGCACCCTGACTCTGAGCAAAGCAGACTACGAGAAGCACAAGGTCTACG
CCTGCGAAGTCACCCATCAGGGCCTGAGTTCCCCTGTCACAAAGAGCTTCA
ACCGGGGAGAGTGTTGATGATATC.

In one embodiment, the vector as described hereinabove is comprised in an isolated host cell. Said host cell may be used for the recombinant production of the antibodies (or fragments thereof) for use in the present invention. In an embodiment, host cells may be prokaryote, yeast, or eukaryote cells preferably mammalian cells, such as, for example: monkey kidney CV1 line transformed by SV40 (COS-7, ATCC CRL 1651); human embryonic kidney line (293 or 293 cells subcloned for growth in suspension culture); baby hamster kidney cells (BHK, ATCC CCL 10); Chinese hamster ovary cells/- DHFR (CHO); mouse Sertoli cells (TM4); mouse myeloma cells SP2/0-AG14 (ATCC CRL 1581 ; ATCC CRL 8287) or NSO (HPA culture collections no. 85110503); monkey kidney cells (CVl ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells; MRC 5 cells; FS4 cells; and a human hepatoma line (Hep G2), as well as DSM's PERC-6 cell line. Expression vectors suitable for use in each of these host cells are also generally known in the art. It should be noted that the term "host cell" generally refers to a cultured cell line. Whole human beings into which an expression vector encoding an antigen binding protein for use according to the invention has been introduced are explicitly excluded from the definition of a "host cell".

Methods for producing an anti-hGPVI antibody or fragment thereof of the present invention are well known in the art. Examples of suitable methods comprise culturing host cells containing the isolated polynucleotide sequence encoding the anti-hGPVI antibody or fragment thereof for use in the present invention under conditions suitable for expression of the anti-hGPVI antibody or fragment thereof, and recovering the expressed anti-hGPVI antibody or fragment thereof. This recombinant process can be used for large scale production of anti-hGPVI antibodies or fragment thereof for use in the present invention, including monoclonal antibodies intended for *in vivo* therapeutic uses. These processes are available in the art and will be known by the skilled person.

In one embodiment, the protein, in particular the antibody or fragment thereof, binding to hGPVI may be purified by chromatography, preferably by affinity chromatography, more preferably by affinity chromatography on protein L agarose.

Therefore, in one embodiment, the protein, in particular the antibody or fragment thereof, binding to hGPVI comprises a domain for binding protein L (PpL). Methods for transferring PpL-binding activity onto proteins of the invention are described in Muzard et al., Analytical Biochemistry 388, 331-338, 2009 and in Lakhrif et al., MAbs. 2016;8(2):379-88, which are incorporated herein by reference.

Another object of the invention is a composition for treating or for use in treating a GPVI-related condition in a subject in need thereof, wherein said composition comprises or consists essentially of at least one inhibitor of the GPVI signaling pathway, in particular at least one isolated humanized protein binding to human Glycoprotein VI (hGPVI), as described herein, wherein said subject presents at least one of the following conditions:
i) said subject has a large ischemic core volume, and/or
ii) said subject has a good cerebral collateral circulation.

As used herein, **"consisting essentially of",** with reference to a composition, pharmaceutical composition, or medicament, means that the at least one inhibitor of the GPVI signaling pathway as described herein is the only therapeutic agent or agent with a biologic activity within said composition, pharmaceutical composition, or medicament.

Another object of the invention is a pharmaceutical composition for treating or for use in treating a GPVI-related condition in a subject in need thereof, wherein said pharmaceutical composition comprises or consists essentially of at least one inhibitor of the GPVI signaling pathway, in particular at least one isolated humanized protein binding to human Glycoprotein VI (hGPVI), as described herein and at least one pharmaceutically acceptable excipient, wherein said subject presents at least one of the following conditions:
i) said subject has a large ischemic core volume, and/or
ii) said subject has a good cerebral collateral circulation.

Pharmaceutically acceptable excipients that may be used in these compositions include, but are not limited to, ion exchangers, alumina, aluminum stearate, lecithin, serum proteins, such as human serum albumin, buffer substances such as acetates, citrates, phosphates, glycine, sorbic acid, potassium sorbate, sugars such as glucose, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

In one embodiment, the pH of the pharmaceutical composition is comprised between 4 to 6 and preferably is of about 5.0. In one embodiment, the pharmaceutical composition comprises PBS pH 7.2-7.7. In one embodiment, the pharmaceutical composition comprises a sodium citrate buffer 20 mM, NaCl 130 mM, pH 5.0.

Another object of the invention is a medicament for treating or for use in treating a GPVI-related condition in a subject in need thereof, wherein said medicament comprises or consists essentially of at least one inhibitor of the GPVI signaling pathway, in particular at least one isolated humanized protein binding to human Glycoprotein VI (hGPVI), as described herein and at least one pharmaceutically acceptable excipient, wherein said subject presents at least one of the following conditions:
i) said subject has a large ischemic core volume, and/or
ii) said subject has a good cerebral collateral circulation.

Another object of the invention is the use of at least one inhibitor of GPVI signaling pathway, in particular at least one isolated humanized protein binding to human Glycoprotein VI (hGPVI), as described herein in the manufacture of a medicament for treating a GPVI-related condition in a subject in need thereof, wherein said subject presents at least one of the following conditions:
i) said subject has a large ischemic core volume, and/or
ii) said subject has a good cerebral collateral circulation.

In one embodiment, the composition, pharmaceutical composition or medicament for use in the present invention further comprises another agent useful for treating a GPVI-related condition, such as, for example a thrombolytic agent.

As used herein, thrombolytic agents are agents capable of dissolving a clot (or thrombus) formed in blood vessels. Examples of thrombolytic agents, include, without limitation, anistreplase (e.g., Eminase); tissue-type plasminogen activator (t-PA) and modified forms of t-PA; streptokinase (e.g., Streptase); urokinase, pro-urokinase and modified forms thereof (e.g., Abbokinase, Kinlytic, TS-01); small molecule plasminogen activator (e.g., TMS-007; SMTP-7; BIIB131); and N-Acetyl Cysteine (NAC).

In one embodiment, the composition, pharmaceutical composition or medicament for use in the present invention further comprises another agent useful for treating a GPVI-related condition, such as, for example an antiplatelet agent.

Examples of antiplatelet agents, include, without limitation, irreversible cyclooxygenase inhibitors such as, for example, Aspirin (Acetylsalicylic acid (ASA)), indobufen (Ibustrin) or Triflusal (Disgren); Adenosine diphosphate (ADP) receptor inhibitors such as, for example, Cangrelor (Kengreal), Clopidogrel (Plavix), Prasugrel (Effient), Ticagrelor (Brilinta) or Ticlopidine (Ticlid); Phosphodiesterase inhibitors such as, for example, Cilostazol (Pletaal); Protease-activated receptor-1 (PAR-1) antagonists such as, for example, Vorapaxar (Zontivity); Glycoprotein IIB/IIIA inhibitors such as, for example, Abciximab (ReoPro), Eptifibatide (Integrilin) or Tirofiban (Aggrastat); Prostacycline analogs such as, for example, Iloprost (Ilomedine), Epoprostenol (Veletri) or Treprostinil (Remodulin); Prostacycline receptor inhibitors such as, for example, Selexipag (Uptravi); Adenosine reuptake inhibitors such as, for example, Dipyridamole (Persantine); Thromboxane inhibitors, Thromboxane synthase inhibitors and Thromboxane receptor antagonists such as, for example, Terutroban.

In one embodiment, the composition, pharmaceutical composition or medicament for use in the present invention further comprises another agent useful for treating a GPVI-related condition, such as, for example, an anticoagulant agent.

Examples of anticoagulant agents, include, without limitation, vitamin K antagonists including coumarin derivatives, such as warfarin (Coumadin), Fluindione (Previscan), Phenprocoumone or acenocoumarol; heparin and derivatives thereof including low molecular weight heparin (LMWH), such as, for example Enoxaparin, Nadroparin, Tinzaparin or Dalteparin; synthetic pentasaccharide inhibitors of factor Xa, such as, for example, Fondaparinux, Idraparinux or Idrabiotaparinux; directly acting oral anticoagulants (DOACs) including direct factor Xa inhibitors (*e.g.,* rivaroxaban, apixaban, edoxaban, betrixaban, darexaban, letaxaban, and eribaxaban) and direct thrombin inhibitors (*e.g,* hirudin, lepirudin, bivalirudin; argatroban and dabigatran); antithrombin protein therapeutics; and other agents such as, for example, Batroxobin; Hementin and Vitamin E.

In one embodiment, the composition, pharmaceutical composition or medicament for use in the present invention further comprises at least one agent, such as, for example, 1, 2 or 3 agents, selected from the group comprising or consisting of a thrombolytic agent, an antiplatelet agent, and an anticoagulant agent.

Another object of the invention is a kit of parts for treating or for use in treating a GPVI-related condition in a subject in need thereof, the kit of parts comprising, in a first part, at least one inhibitor of GPVI signaling pathway, in particular at least one isolated humanized protein binding to human Glycoprotein VI (hGPVI) as described hereinabove, preferably at least one isolated anti-hGPVI antibody or a fragment thereof as described herein and, in a second part, at least one agent, such as, for example, 1, 2 or 3 agents, selected from the group comprising or consisting of a thrombolytic agent, an antiplatelet agent, and an anticoagulant agent, wherein said subject presents at least one of the following conditions:
i) said subject has a large ischemic core volume, and/or
ii) said subject has a good cerebral collateral circulation.

In one embodiment, the inhibitor, in particular the protein, as described herein inhibits GPVI signaling and/or signaling of a ligand of GPVI.

In one embodiment, the inhibitor, in particular the protein, as described herein is an anti-hGPVI antibody or fragment thereof that inhibits GPVI signaling and/or signaling of a ligand of GPVI. As used herein, the term "inhibit" means that the inhibitor, in particular the protein, as described herein is capable of blocking, reducing, preventing or neutralizing GPVI interaction with its ligands, GPVI signaling and/or the activation of molecules from the GPVI signaling pathway.

Examples of ligands of GPVI are provided hereinabove.

In one embodiment, the inhibitor, in particular the protein, as described herein is a neutralizing anti-hGPVI antibody or fragment thereof.

In one embodiment, the inhibitor, in particular the protein, as described herein inhibits the binding of GPVI to a ligand thereof (such as, for example, collagen, fibrin or any other GPVI ligand capable of inducing downstream signaling and platelet activation).

In one embodiment, the inhibitor, in particular the protein, as described herein inhibits and/or prevents platelet activation, secretion and aggregation in response to a ligand of GPVI, such as, for example, collagen. In an embodiment, the inhibitor, in particular the protein, as described herein inhibits and/or prevents platelet adhesion to a ligand of GPVI, such as, for example, collagen.

In one embodiment, the inhibitor, in particular the protein, as described herein inhibits and/or prevents GPVI-dependent thrombin production in response to a ligand of GPVI, such as, for example, fibrin. In an embodiment, the inhibitor, in particular the protein, as described herein inhibits and/or prevent platelet-catalyzed thrombin production in response to collagen and/or to tissue factor.

In one embodiment, the inhibitor, in particular the protein, as described herein inhibits and/or prevents platelet recruitment by a ligand of GPVI (such as, for example, fibrin) via GPVI.

In one embodiment, the inhibitor, in particular the protein, as described herein induces saturation of platelets in whole blood or in platelet rich plasma when present at a concentration ranging from about 1 to about 200 µg/mL, preferably from about 2 to about 100 µg/mL, and more preferably from about 5 to about 50 µg/mL.

In one embodiment, the inhibitor, in particular the protein, as described herein inhibits collagen-induced platelet aggregation when used at a concentration of at least about 15 µg/mL, preferably of at least about 10 µg/mL. Preferably, the inhibitor, in particular the protein, as described herein fully inhibits collagen-induced platelet aggregation when used at such concentrations.

In one embodiment, the IC50 of the inhibitor, in particular the protein, as described herein for inhibiting collagen-induced platelet aggregation ranges from about 0.5 to about 10 µg/mL, preferably from about 1 to about 6 µg/mL, more preferably from about 2 to about 3.2 µg/mL.

In one embodiment, the concentration of the inhibitor, in particular the protein, as described herein reducing by 50% the velocity of collagen-induced platelet aggregation ranges from about 0.5 to about 5 µg/mL, preferably from about 1 to about 3 µg/mL, more preferably of about 2 µg/mL.

In one embodiment, the concentration of the inhibitor, in particular the protein, as described herein reducing the intensity of collagen-induced platelet aggregation ranges from about 0.5 to about 10 µg/mL, preferably from about 1 to about 6 µg/mL, more preferably of about 3.2 µg/mL.

In one embodiment, the inhibitor, in particular the protein, as described herein does not induce depletion of GPVI when administered in vivo, such as, for example, when administered at a dose ranging from 0.01 to 1000 mg.

In one embodiment, the inhibitor, in particular the protein, as described herein does not induce a decrease in platelet count, i.e., thrombocytopenia, when administered in vivo, such as, for example, when administered at a dose ranging from 0.01 to 1000 mg.

In one embodiment, the inhibitor, in particular the protein, as described herein does not induce an increase in bleeding time.

In one embodiment, the inhibitor, in particular the protein, as described herein decreases the incidence of the intracranial hemorrhages (ICHs) or prevents the occurrence of ICHs in a subject affected by, preferably diagnosed with a GPVI-related condition. In one embodiment, said intracranial hemorrhages are symptomatic ICHs. In one embodiment, said intracranial hemorrhages are non-symptomatic ICHs.

In one embodiment, the inhibitor, in particular the protein, as described herein prevents death, or decreases the risk of death in a subject affected by, preferably diagnosed with a GPVI-related condition. In one embodiment, the death is a non-stroke-related death. In one embodiment, the death is a stroke-related death, such as, for example, worsening of the initial stroke, ICHs or cerebral herniation.

In one embodiment, the inhibitor, in particular the protein, as described herein decreases the degree of disability or dependence of a subject following a GPVI-related condition, preferably a stroke, in particular at long-term, such as, for example, 90 days after the administration of said inhibitor, in particular said protein. In one embodiment, the inhibitor, in particular the protein, as described herein is for preventing disability or dependence, or for limiting the extent of disability or dependence, of a subject following a GPVI-related condition, preferably a stroke, in particular at long-term, such as, for example, 90 days after the administration of said inhibitor, in particular said protein
Means for evaluating disability or dependence following a GPVI-related condition, in particular a stroke, are well-known by the skilled artisan in the art, and include for example, the modified Rankin Scale.

The mRS is a commonly used scale for measuring the degree of disability or dependence in the daily activities of people who have suffered a stroke or other causes of neurological disability. The scale runs from 0-6 (see Table 1), running from perfect health without symptoms to death.

**Table 1**

| **Score** | **Symptoms** |
|---|---|
| 0 | No symptoms |
| 1 | No significant disability. Able to carry out all usual activities, despite some symptoms. |
| 2 | Slight disability. Able to look after own affairs without assistance, but unable to carry out all previous activities. |
| 3 | Moderate disability. Requires some help, but able to walk unassisted. |
| 4 | Moderately severe disability. Unable to attend to own bodily needs without assistance, and unable to walk unassisted. |
| 5 | Severe disability. Requires constant nursing care and attention, bedridden, incontinent. |
| 6 | Dead. |

In one embodiment, the inhibitor, in particular the protein, as described herein is for preventing disability or dependance associated with a mRS score of 5 or more. In one embodiment, the inhibitor, in particular the protein, as described herein is for preventing disability or dependence associated with a mRS score of 4 or more, or for preventing disability or dependance associated with a mRS score of 3 or more, in a subject following a GPVI-related condition, preferably a stroke, in particular at long-term, such as, for example, 90 days after the administration of said inhibitor.

In one embodiment, the inhibitor, in particular the protein, as described herein is for decreasing the proportion of subjects associated with a mRS score of 4 or more following a GPVI-related condition, preferably a stroke, in particular at long-term, such as, for example, 90 days after the administration of said inhibitor. In one embodiment, the inhibitor, in particular the protein, as described herein is for increasing the proportion of subjects associated with a mRS score of 2 or less following a GPVI-related condition, preferably a stroke, in particular at long-term, such as, for example, 90 days after the administration of said inhibitor.

In one embodiment, the inhibitor, in particular the protein, as described herein decreases the neurological symptoms of a subject affected with a GPVI-related condition, preferably a stroke, in particular at short term, such as, for example, at 6 hours, 12 hours, 24 hours, 36 hours, 48 hours or more after administration of said inhibitor, in particular said protein.

Means for evaluating neurological symptoms of a subject affected with a GPVI-related condition, in particular a stroke, are well-known by the skilled artisan in the art, and include for example, NIH stroke scale (NIHSS) as described hereinbelow.

The NIHSS is composed of 11 items (see Table 2 below), each of which scores a specific ability between 0-2, 0-3 or 0-4. For each item, a score of 0 typically indicates normal function in that specific ability, while a higher score is indicative of some level of impairment. The individual scores from each item are summed in order to calculate a patient's total NIHSS score. The maximum possible score is 42, with the minimum score being 0.

**Table 2**

| **Instructions** | **Scale definition** |
|---|---|
| **1a. Level of Consciousness:** | 0 = Alert; keenly responsive. |
| The investigator must choose a response if a full evaluation is prevented by such obstacles as an endotracheal tube, language barrier, orotracheal trauma/bandages. A 3 is scored only if the patient makes no movement (other than reflexive posturing) in response to noxious stimulation | 1 = Not alert; but arousable by minor stimulation to obey, answer, or respond. |
| | 2 = Not alert; requires repeated stimulation to attend, or is obtunded and requires strong or painful stimulation to make movements (not stereotyped). |
| | 3 = Responds only with reflex motor or autonomic effects or totally unresponsive, flaccid, and areflexic. |
| **1b. LOC Questions:** | 0 = Answers both questions correctly. |
| The patient is asked the month and his/her age. The answer must be correct - there is no partial credit for being close. Aphasic and stuporous patients who do not comprehend the questions will score 2. Patients unable to speak because of endotracheal intubation, orotracheal trauma, severe dysarthria from any cause, language barrier, or any other problem not secondary to aphasia are given a 1. It is important that only the initial answer be graded and that the examiner not "help" the patient with verbal or non-verbal cues. | |
| | 1 = Answers one question correctly. |
| | 2 = Answers neither question correctly. |
| **1c. LOC Commands:** | 0 = Performs both tasks correctly. |
| The patient is asked to open and close the eyes and then to grip and release the non-paretic hand. Substitute another one step command if the hands cannot be used. Credit is given if an unequivocal attempt is made but not completed due to weakness. If the patient does not respond to command, the task should be demonstrated to him or her (pantomime), and the result scored (i.e., follows none, one or two commands). Patients with trauma, amputation, or other physical impediments should be given suitable one-step commands. Only the first attempt is scored. | 1 = Performs one task correctly. |
| | 2 = Performs neither task correctly. |
| **2. Best Gaze:** | 0 = Normal. |
| Only horizontal eye movements will be tested. Voluntary or reflexive (oculocephalic) eye movements will be scored, but caloric testing is not done. If the patient has a conjugate deviation of the eyes that can be overcome by voluntary or reflexive activity, the score will be 1. If a patient has an isolated peripheral nerve paresis (CN III, IV or VI), score a 1. Gaze is testable in all aphasic patients. Patients with ocular trauma, bandages, pre-existing blindness, or other disorder of visual acuity or fields should be tested with reflexive movements, and a choice made by the investigator. Establishing eye contact and then moving about the patient from side to side will occasionally clarify the presence of a partial gaze palsy. | 1 = Partial gaze palsy; gaze is abnormal in one or both eyes, but forced deviation or total gaze paresis is not present. |
| | 2 = Forced deviation, or total gaze paresis not overcome by the oculocephalic maneuver |
| **3. Visual:** | 0 = No visual loss. |
| Visual fields (upper and lower quadrants) are tested by confrontation, using finger counting or visual threat, as appropriate. Patients may be encouraged, but if they look at the side of the moving fingers appropriately, this can be scored as normal. If there is unilateral blindness or enucleation, visual fields in the remaining eye are scored. Score 1 only if a clear-cut asymmetry, including quadrantanopia, is found. If patient is blind from any cause, score 3. Double simultaneous stimulation is performed at this point. If there is extinction, patient receives a 1, and the results are used to respond to item 11. | 1 = Partial hemianopia. |
| | 2 = Complete hemianopia. |
| | 3 = Bilateral hemianopia (blind including cortical blindness). |
| **4. Facial Palsy:** | 0 = Normal symmetrical movements. |
| Ask - or use pantomime to encourage - the patient to show teeth or raise eyebrows and close eyes. Score symmetry of grimace in response to noxious stimuli in the poorly responsive or noncomprehending patient. If facial trauma/bandages, orotracheal tube, tape or other physical barriers obscure the face, these should be removed to the extent possible. | |
| | 1 = Minor paralysis (flattened nasolabial fold, asymmetry on smiling). |
| | 2 = Partial paralysis (total or neartotal paralysis of lower face). |
| | 3 = Complete paralysis of one or both sides (absence of facial movement in the upper and lower face). |
| **5. Motor Arm:** | 0 = No drift; limb holds 90 (or 45) degrees for full 10 seconds. |
| The limb is placed in the appropriate position: extend the arms (palms down) 90 degrees (if sitting) or 45 degrees (if supine). Drift is scored if the arm falls before 10 seconds. The aphasic patient is encouraged using urgency in the voice and pantomime, but not noxious stimulation. Each limb is tested in turn, beginning with the non-paretic arm. Only in the case of amputation or joint fusion at the shoulder, the examiner should record the score as untestable (UN), and clearly write the explanation for this choice. | |
| | 1 = Drift; limb holds 90 (or 45) degrees, but drifts down before full 10 seconds; does not hit bed or other support. |
| | 2 = Some effort against gravity; limb cannot get to or maintain (if cued) 90 (or 45) degrees, drifts down to bed, but has some effort against gravity. |
| | 3 = No effort against gravity; limb falls. |
| | 4 = No movement. UN = Amputation or joint fusion, explain: |
| | 5a. Left Arm |
| | 5b. Right Arm |
| **6. Motor Leg:** | 0 = No drift; leg holds 30-degree position for full 5 seconds. |
| The limb is placed in the appropriate position: hold the leg at 30 degrees (always tested supine). Drift is scored if the leg falls before 5 seconds. The aphasic patient is encouraged using urgency in the voice and pantomime, but not noxious stimulation. Each limb is tested in turn, beginning with the non-paretic leg. Only in the case of amputation or joint fusion at the hip, the examiner should record the score as untestable (UN), and clearly write the explanation for this choice. | |
| | 1 = Drift; leg falls by the end of the 5-second period but does not hit bed. |
| | 2 = Some effort against gravity; leg falls to bed by 5 seconds, but has some effort against gravity. |
| | 3 = No effort against gravity; leg falls to bed immediately. |
| | 4 = No movement. |
| | UN = Amputation or joint fusion, explain: |
| | _ 6a. Left Leg |
| | 6b. Right Leg |
| **7. Limb Ataxia:** | 0 = Absent. |
| This item is aimed at finding evidence of a unilateral cerebellar lesion. Test with eyes open. In case of visual defect, ensure testing is done in intact visual field. The finger-nose-finger and heel-shin tests are performed on both sides, and ataxia is scored only if present out of proportion to weakness. Ataxia is absent in the patient who cannot understand or is paralyzed. Only in the case of amputation or joint fusion, the examiner should record the score as untestable (UN), and clearly write the explanation for this choice. In case of blindness, test by having the patient touch nose from extended arm position. | 1 = Present in one limb. |
| | 2 = Present in two limbs. UN = Amputation or joint fusion, explain: |
| **8. Sensory:** | 0 = Normal; no sensory loss. |
| Sensation or grimace to pinprick when tested, or withdrawal from noxious stimulus in the obtunded or aphasic patient. Only sensory loss attributed to stroke is scored as abnormal and the examiner should test as many body areas (arms [not hands], legs, trunk, face) as needed to accurately check for hemisensory loss. A score of 2, "severe or total sensory loss," should only be given when a severe or total loss of sensation can be clearly demonstrated. Stuporous and aphasic patients will, therefore, probably score 1 or 0. The patient with brainstem stroke who has bilateral loss of sensation is scored 2. If the patient does not respond and is quadriplegic, score 2. Patients in a coma (item 1a=3) are automatically given a 2 on this item. | 1 = Mild-to-moderate sensory loss; patient feels pinprick is less sharp or is dull on the affected side; or there is a loss of superficial pain with pinprick, but patient is aware of being touched. |
| | 2 = Severe to total sensory loss; patient is not aware of being touched in the face, arm, and leg. |
| **9. Best Language:** | 0 = No aphasia; normal. |
| A great deal of information about comprehension will be obtained during the preceding sections of the examination. For this scale item, the patient is asked to describe what is happening in the attached picture, to name the items on the attached naming sheet and to read from the attached list of sentences. Comprehension is judged from responses here, as well as to all of the commands in the preceding general neurological exam. If visual loss interferes with the tests, ask the patient to identify objects placed in the hand, repeat, and produce speech. The intubated patient should be asked to write. The patient in a coma (item 1a=3) will automatically score 3 on this item. The examiner must choose a score for the patient with stupor or limited cooperation, but a score of 3 should be used only if the patient is mute and follows no one-step commands. | 1 = Mild-to-moderate aphasia; some obvious loss of fluency or facility of comprehension, without significant limitation on ideas expressed or form of expression. Reduction of speech and/or comprehension, however, makes conversation about provided materials difficult or impossible. For example, in conversation about provided materials, examiner can identify picture or naming card content from patient's response. |
| | 2 = Severe aphasia; all communication is through fragmentary expression; great need for inference, questioning, and guessing by the listener. Range of information that can be exchanged is limited; listener carries burden of communication. Examiner cannot identify materials provided from patient response. |
| | 3 = Mute, global aphasia; no usable speech or auditory comprehension. |
| **10. Dysarthria:** | 0 = Normal. |
| If patient is thought to be normal, an adequate sample of speech must be obtained by asking patient to read or repeat words from the attached list. If the patient has severe aphasia, the clarity of articulation of spontaneous speech can be rated. Only if the patient is intubated or has other physical barriers to producing speech, the examiner should record the score as untestable (UN), and clearly write an explanation for this choice. Do not tell the patient why he or she is being tested. | 1 = Mild-to-moderate dysarthria; patient slurs at least some words and, at worst, can be understood with some difficulty. |
| | 2 = Severe dysarthria; patient's speech is so slurred as to be unintelligible in the absence of or out of proportion to any dysphasia, or is mute/anarthric. |
| | UN = Intubated or other physical barrier, |
| | explain: |
| **11. Extinction and Inattention (formerly Neglect):** | 0 = No abnormality. |
| | 1 = Visual, tactile, auditory, spatial, or personal inattention or extinction to bilateral simultaneous stimulation in one of the sensory modalities. |
| Sufficient information to identify neglect may be obtained during the prior testing. If the patient has a severe visual loss preventing visual double simultaneous stimulation, and the cutaneous stimuli are normal, the score is normal. If the patient has aphasia but does appear to attend to both sides, the score is normal. The presence of visual spatial neglect or anosagnosia may also be taken as evidence of abnormality. Since the abnormality is scored only if present, the item is never untestable. | |
| | 2 = Profound hemi-inattention or extinction to more than one modality; does not recognize own hand or orients to only one side of space. |

Then, a score is associated with the stroke severity, as presented in the Table 3 below.

**Table 3**

| **Score** | **Stroke severity** |
|---|---|
| 0 | No stroke symptoms |
| 1-4 | Minor stroke |
| 5-15 | Moderate stroke |
| 16-20 | Moderate to severe stroke |
| 21-42 | Severe stroke |

In one embodiment, the inhibitor, in particular the protein, as described herein increases the probability of having a complete recanalization following thrombectomy (and thrombolysis).

In one embodiment, the inhibitor, in particular the protein, as described herein protects the cerebral tissue following the GPVI-related condition. Thus, the present invention also relates to a method for protecting the cerebral tissue following the GPVI-related condition, comprising administration to a subject in need thereof an inhibitor, in particular a protein, as described herein.

In one embodiment, the inhibitor, in particular the protein, as described herein decreases the volume of infarct, the volume of hemorrhagic transformation and/or the volume of ischemic injury following the GPVI-related condition. Thus, the present invention also relates to a method for decreasing the volume of infarct, the volume of hemorrhagic transformation and/or the volume of ischemic injury following the GPVI-related condition, comprising administration to a subject in need thereof an inhibitor, in particular a protein, as described herein.

Examples of GPVI-related conditions are well known in the art and include, without limitation, inflammation, thrombosis, disorders associated with abnormal or aberrant megakaryocyte and/or platelet proliferation, differentiation, morphology, migration, aggregation, degranulation and/or function, thrombotic disorders (such as, for example, thrombotic occlusion of coronary arteries), diseases exhibiting quantitative or qualitative platelet dysfunction and diseases displaying endothelial dysfunction, cerebral vascular diseases, coronary diseases, disorders resulting from any blood vessel insult that can result in platelet aggregation, disorders associated with aberrant signal transduction in response to ligands of GPVI, disorders associated with aberrant levels of GPVI expression and/or activity either in cells that normally express GPVI or in cells that do not express GPVI, bone marrow and peripheral blood or disorders in which platelets contribute by modulating inflammatory responses.

In one embodiment, the GPVI-related condition is a cardiovascular disease or event.

In one embodiment, the GPVI-related condition is selected from the group comprising or consisting of venous thrombosis, restenosis, acute coronary syndrome, cerebrovascular accidents due to atherosclerosis, myocardial infarction (heart attack), pulmonary embolism, critical limb ischemia, peripheral artery disease, arterial thrombosis including atherothrombosis, ischemic events, acute cerebrovascular ischemia (stroke), percutaneous coronary intervention, stenting thrombosis, ischemia (acute and chronic), diseases of the aorta and its branches (such as aortic aneurysm, thrombosis), acute phlebitis, pulmonary embolism, cancer-associated thrombosis (Trousseau syndrome), inflammatory thrombosis and thrombosis associated to infection, coronary artery diseases, cerebral artery diseases, cerebral vascular diseases or events including ischemic stroke, venous thromboembolism diseases (such as, for example, diseases involving leg swelling, pain and ulceration, pulmonary embolism, abdominal venous thrombosis), thrombotic microangiopathies, and vascular purpura.

In one embodiment, the inhibitor, protein, composition, pharmaceutical composition, medicament or kit as described herein is used to modulate leukocyte-platelet in inflammation and/or thrombosis. Therefore, according to an embodiment, the inhibitor, protein, composition, pharmaceutical composition or medicament as described herein is used to treat inflammation and/or thrombosis. In one embodiment, the inhibitor, protein, composition, pharmaceutical composition or medicament as described hereinabove is used to treat thrombo-inflammation.

In one embodiment, the inhibitor, protein, composition, pharmaceutical composition, medicament or kit as described herein is used to modulate, preferably to prevent, platelet adhesion aggregation and degranulation.

In one embodiment, the inhibitor, protein, composition, pharmaceutical composition, medicament or kit as described herein is used to treat thrombotic disorders (such as, for example, thrombotic occlusion of coronary arteries), diseases exhibiting quantitative or qualitative platelet dysfunction and diseases displaying endothelial dysfunction.

In one embodiment, the inhibitor, protein, composition, pharmaceutical composition, medicament or kit as described herein is used to treat stroke, preferably an ischemic stroke. In one embodiment, said stroke is an acute stroke. In one embodiment, the GPVI-related disease or condition is an acute ischemic stroke.

In one embodiment, the inhibitor, protein, composition, pharmaceutical composition, medicament or kit as described herein is used to treat coronary diseases (such as, for example, cardiovascular diseases including unstable angina pectoris, myocardial infarction, acute myocardial infarction, coronary artery disease, coronary revascularization, coronary restenosis, ventricular thromboembolism, atherosclerosis, coronary artery disease (e. g., arterial occlusive disorders), plaque formation, cardiac ischemia, including complications related to coronary procedures, such as percutaneous coronary artery angioplasty (balloon angioplasty) procedures). With respect to coronary procedures, such treatment can be achieved via administration of an inhibitor, a protein, a composition, a pharmaceutical composition or a medicament as described above prior to, during, or subsequent to the procedure. In a preferred embodiment, such administration can be utilized to prevent acute cardiac ischemia following angioplasty.

In one embodiment, the inhibitor, protein, composition, pharmaceutical composition, medicament or kit as described herein is used to treat disorders resulting from any blood vessel insult that can result in platelet aggregation. Such blood vessel insults include, but are not limited to, vessel wall injury, such as vessel injuries that result in a highly thrombogenic surface exposed within an otherwise intact blood vessel e. g., vessel wall injuries that result in release of ADP, thrombin and/or epinephrine, fluid shear stress that occurs at the site of vessel narrowing, ruptures and/or tears at the sites of atherosclerotic plaques, and injury resulting from balloon angioplasty or atherectomy.

Further, in certain embodiments, it is preferred that the inhibitor, in particular the protein, as described herein does not affect other platelet attributes or functions, such as agonist-induced platelet shape change (e.g., GPIb-vWF-mediated platelet activation), release of internal platelet granule components, activation of signal transduction pathways or induction of calcium mobilization upon platelet activation by agonists that do not interact with GPVI.

In one embodiment, the inhibitor, protein, composition, pharmaceutical composition, medicament or kit as described herein is used to treat disorders associated with aberrant signal transduction in response to ligands of GPVI (including, without limitation, collagen, fibrin, fibronectin, vitronectin and laminins) or to other extracellular matrix proteins.

In one embodiment, the inhibitor, protein, composition, pharmaceutical composition medicament or kit as described herein is used to treat disorders associated with aberrant levels of GPVI expression and/or activity either in cells that normally express GPVI or in cells that do not express GPVI. For example, the inhibitor, in particular the protein, of the invention can be used to modulate disorders associated with aberrant expression of GPVI in cancerous (e. g., tumor) cells that do not normally express GPVI. Such disorders can include, for example, ones associated with tumor cell migration and progression to metastasis.

In one embodiment, the inhibitor, protein, composition, pharmaceutical composition, medicament or kit as described herein is used to modulate immunoregulatory functions of platelets.

In one embodiment, the inhibitor, protein, composition, pharmaceutical composition, medicament or kit as described herein is used to treat disorders of bone marrow and peripheral blood.

In one embodiment, the inhibitor, protein, composition, pharmaceutical composition, medicament or kit as described herein is used to treat disorders in which platelets contribute by modulating inflammatory responses including, without limitation, sustained or prolonged inflammation associated with infection, arthritis, fibrosis or disorders in which platelets modulate cell functions including, without limitation, cancer cells proliferation and/or dissemination.

In one embodiment, the subject is a male. In one embodiment, the subject is a female.

In one embodiment, the subject is a child, *i.e*., younger than 18 years old. In one embodiment, the subject is an adult, *i.e*., 18 years old or older.

In one embodiment, the subject is of or older than 50, 55, 60, 65, 70, 75, 80, 85, 90, 95 years of age or more. In one embodiment, the subject is of 65 years of age or older. In one embodiment, the subject is of 80 years of age or older.

In one embodiment, the subject has a large ischemic core volume. In one embodiment, the subject has a large ischemic core volume at baseline *(i.e.* before the administration of the inhibitor, protein, composition, pharmaceutical composition, medicament or kit as described herein). As used herein, the ischemic core refers to the irreversibly injured brain tissue.

In one embodiment, the ischemic core volume is assessed by measuring the acute infarct volume (AIV) with magnetic resonance imaging (MRI), and/or computed tomography (CT), including CT perfusion (CTP), non-contrast computed tomography (NCCT) and computed tomography angiography (CTA).

In one embodiment, the AIV is measured using apparent diffusion coefficient (ADC) with MRI. In one embodiment, the AIV is measured using an ADC threshold of 620×10-6mm2s-1. As used herein, ADC is a measure of the magnitude of diffusion (of water molecules) within tissue.

In one embodiment, the AIV is measured using the relative cerebral blood flow (rCBF) with CT. In one embodiment, the AIV is measured using a rCBF threshold of 30% with CT. As used herein, CBF is defined as the volume of blood that passes through a specific quantity of brain tissue during a particular period of time.

In one embodiment, a large ischemic core volume is an ischemic core volume equals or above 5, 6, 7, 8, 9 or 10 mL. Thus, in one embodiment, the subject with a large ischemic core volume has an ischemic core volume equals or above 5, 6, 7, 8, 9 or 10 mL. In one specific embodiment, the subject with a large ischemic core volume has an ischemic core volume equals or above 7 mL.

In one embodiment, a large ischemic core volume is an ischemic core with a volume ranging from 5 to 200 mL, preferably ranging from 6 to 200 mL, more preferably ranging from 7 to 200 mL.

In one embodiment, the subject has a good cerebral collateral circulation. In one embodiment, the subject has a good cerebral collateral circulation at baseline *(i.e.* before the administration of the inhibitor, protein, composition, pharmaceutical composition, medicament or kit as described herein). As used herein, the cerebral collateral circulation refers to the alternative network of vascular channels that stabilize cerebral blood flow when principal conduits fail.

In one embodiment, the cerebral collateral circulation is assessed by measuring the Tan Score with computed tomography angiography (CTA).

As used herein, the Tan Score is used to evaluate the status of collateral vessels in ischemic score (Tan et al., "CT angiography clot burden score and collateral score: correlation with clinical and radiologic outcomes in acute middle cerebral artery infarct", AJNR Am J Neuroradiol. 2009 Mar;30(3):525-31). The Tan score has a value ranging from 0 to 3, as defined hereinbelow:
- 0: absent collateral supply to the occluded middle cerebral artery (MCA) territory
- 1: collateral supply filling ≤50% but >0% of the occluded MCA territory
- 2: collateral supply filling >50% but <100% of the occluded MCA territory
- 3: 100% collateral supply of the occluded MCA territory.

In one embodiment, the subject with a good cerebral collateral circulation has a Tan score value of 2 or 3, preferably wherein said Tan score is measured with CTA.

In one embodiment, the cerebral collateral circulation is assessed by measuring hypoperfusion intensity ratio (HIR) with CTP or magnetic resonance imaging perfusion (MRP).

As used herein, the HIR refers to the ratio of the volume of tissue with a time-to-maximum (Tmax) of > 10 seconds divided by the volume of tissue with a Tmax of >6 seconds (Lyndon et al., "Hypoperfusion Intensity Ratio Correlates with CTA Collateral Status in Large-Vessel Occlusion Acute Ischemic Stroke", AJNR Am J Neuroradiol. 2021 Aug; 42(8): 1380-1386).

In one embodiment, the subject has undergone, is undergoing, or will undergo endovascular treatment, such as, for example, thrombectomy or embolectomy. As used herein, the term "thrombectomy" and "mechanical thrombectomy" are equivalent and refer to a mechanical interventional procedure by which a thrombus is removed.

In one embodiment, the subject has undergone, is undergoing, or will undergo a thrombectomy.

In one embodiment, the subject was treated, is treated or is to be treated with a thrombolytic agent, preferably wherein said thrombolytic agent is as described hereinabove. In one embodiment, said thrombolytic agent is t-PA. In one embodiment, the subject was treated, is treated or is to be treated with a thrombolytic agent but has not undergone, is not undergoing, or will not undergo thrombectomy.

In one embodiment, the subj ect was treated with a thrombolytic agent, preferably t-PA, and received the thrombolytic agent less than about 2 hours after stroke.

In one embodiment, the subject was treated, is treated or is to be treated with an antiplatelet agent, preferably wherein said antiplatelet agent is as described hereinabove.

In one embodiment, the subject was treated, is treated or is to be treated with an anticoagulant agent, preferably wherein said antiplatelet agent is as described hereinabove.

In one embodiment, the thrombolytic agent, the antiplatelet agent and/or the anticoagulant agent is/are to be administered before, concomitantly or after the administration of the inhibitor, in particular the protein (preferably the antibody or fragment thereof), of the present invention.

In one embodiment, the subject experienced a GPVI-related condition, such as, for example, a cardiovascular event (e.g., thrombosis, stroke, myocardial infarction or a cerebrovascular accident) less than 48 hours, preferably less than 24 hours, 12 hours, 10 hours, 8 hours, 6 hours, 4 hours or less, before the administration of the inhibitor, in particular the protein, as described herein (and optionally before the administration of the thrombolytic agent).

In one embodiment, the inhibitor, protein, composition, pharmaceutical composition, medicament or kit as described herein is administered during the first 24 hours, preferably the first 12 hours, 10 hours, 8 hours, 6 hours, 4 hours or less after the onset of the GPVI-related condition.

In one embodiment, a dose of the inhibitor, in particular the protein, as described herein ranging from about 0.5 mg/kg to about 50 mg/kg is administered (or is to be administered) to the subject, preferably ranging from about 1 mg/kg to about 32 mg/kg, more preferably of about 8 mg/kg. In another embodiment, a dose of inhibitor, in particular of humanized protein, ranging from about 2.5 mg/kg to about 25 mg/kg, preferably from about 5 mg/kg to about 15 mg/kg, more preferably of about 8 mg/kg is to be administered to the subject. In one embodiment, a dose of the inhibitor, in particular the protein, as described herein of about 0.5 mg/kg, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or of about 50 mg/kg is administered (or is to be administered) to the subject.

In one embodiment, a dose of the inhibitor, in particular the protein, as described herein ranging from about 30 mg to about 3000 mg is administered (or is to be administered) to the subject, preferably ranging from about 60 mg to about 2000 mg, more preferably of about 100 to about 1000 mg, and even more preferably of about 500 mg. In one embodiment, a dose of the inhibitor, in particular the protein, as described herein of about 30, 60, 62.5, 90, 100, 125, 150, 175, 200, 225, 250, 275, 300, 325, 350, 375, 400, 450, 500, 550, 600, 650, 700, 750, 800, 850, 900, 950, 1000, 1500, 2000, 2500 or of about 3000 mg is administered (or is to be administered) to the subject. In another embodiment, a dose of the inhibitor, in particular the protein, as described herein ranges from about 100 mg to about 2000 mg, from about 125 mg to about 2000 mg, preferably from about 250 mg to about 1000 mg or from about 500 mg to about 1000 mg. In one embodiment, a dose of the inhibitor, in particular the protein, as described herein of about 1000 mg is administered (or is to be administered) to the subject.

The composition will be formulated for administration to the subject. The compositions may be administered parenterally, by inhalation spray, rectally, nasally, or via an implanted reservoir. The term administration used herein includes subcutaneous, intravenous, intramuscular, intra-articular, intra-synovial, intrasternal, intrathecal, intrahepatic, intralesional and intracranial injection or infusion techniques.

In one embodiment, the inhibitor, in particular the protein, as described herein is injected, preferably by intravenous infusion. In another embodiment, the inhibitor, in particular the protein, as described herein is injected intraperitoneally. In another embodiment, the inhibitor, in particular the protein, as described herein is injected intradermally.

Examples of forms adapted for injection include, but are not limited to, solutions, such as, for example, sterile aqueous solutions, gels, dispersions, emulsions, suspensions, solid forms suitable for using to prepare solutions or suspensions upon the addition of a liquid prior to use, such as, for example, powder, liposomal forms and the like.

Sterile injectable forms of the compositions may be aqueous or an oleaginous suspension. These suspensions may be formulated according to techniques known in the art using suitable dispersing or wetting agents and suspending agents. The sterile injectable preparation may also be a sterile injectable solution or suspension in a nontoxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

In one embodiment, the inhibitor, in particular the protein, as described herein is continuously administered to the subject during at least 2 hours, preferably during at least 4 to 6 hours (e.g. during about 2 hours, 2.5, 3, 3.5, 4, 4.5, 5, 5.5, 6, 6.5, 7, 7.5, 8, 8.5, 9, 9.5, 10, 10.5, 11, 11.5 or during about 12 hours). As used herein, the terms "continuously administered" refers to the administration of a compound for a prolonged period of time with a substantially constant speed of administration.

In another embodiment, a first bolus of the inhibitor, in particular the protein, as described herein is injected, followed by the continuous administration of the remaining dose of the inhibitor, in particular the protein.

In one embodiment, said first bolus administration comprises about 10 to 50%, preferably about 20% of the total dosage of the inhibitor, in particular the isolated humanized protein, to be administered. In one embodiment, said first bolus administration comprises about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or about 50% of the total dosage of the inhibitor, in particular the isolated humanized protein, to be administered.

In one embodiment, said first bolus is administered in about 5 to 30 minutes, preferably in about 15 minutes.

In one embodiment, about 20% of the total dosage of the inhibitor, in particular the protein, as described herein are administered during the first 15 minutes of the administration, followed by a continuous administration of the remaining 80% during the next 5 hours 45 minutes.

In one embodiment, said specific administration regimen (continued administration during at least 2 hours, with optionally a first bolus) allows a prolonged effect of the inhibitor, in particular the protein, which may be observed after the end of administration of said inhibitor, in particular said protein. In one embodiment, the effect of the inhibitor, in particular the protein, on platelet aggregation is observed for at least about 1, 2, 3, 4, 5, 6, 12, 18, 24, 36 or 48 hours after the end of the administration of the inhibitor, in particular the protein.

Another object of the invention is a method of treating a GPVI-related condition, preferably a stroke, wherein said method comprises administering to a subject in need thereof an inhibitor of the GPVI signaling pathway, in particular a protein, as described herein, or a composition, pharmaceutical composition, medicament or kit as described herein, wherein said subject presents at least one of the following conditions:
i) said subject has a large ischemic core volume, and/or
ii) said subject has a good cerebral collateral circulation.

The embodiments described hereinabove apply *mutatis mutandis* to the methods as described herein.

In one embodiment, the method as described herein comprises administering a therapeutically effective amount of the inhibitor, in particular the protein, to the subject.

In one embodiment, the method as described herein further comprises treating the subject with endovascular treatment such as, for example, a thrombectomy. In one embodiment, said endovascular treatment such as, for example, a thrombectomy, is carried out before, concomitantly or after the administration of the inhibitor, in particular the protein (preferably the antibody or fragment thereof), of the present invention.

In one embodiment, the method as described herein further comprises a step of administering another agent useful for treating a GPVI-related condition, such as, for example a thrombolytic agent, preferably t-PA, an antiplatelet agent and/or an anticoagulant agent. In one embodiment, said additional agent is administered before, concomitantly or after the administration of the inhibitor, in particular the protein (preferably the antibody or fragment thereof), of the present invention.

The present invention further relates to a method for enhancing the potency of a thrombolytic agent (preferably t-PA) for treating a GPVI-related condition in a subject in need thereof, wherein said method comprises administering a combination of said thrombolytic agent with an inhibitor, in particular a protein, of the invention (preferably a therapeutically effective amount of the inhibitor, in particular the protein, of the invention) to the subject, wherein said subject has cardiac history and/or is not affected, or is not diagnosed, with diabetes.

In one embodiment, the method of the invention allows decreasing the dose of thrombolytic agent (preferably t-PA) to be administered to the subject.

The embodiments described hereinabove apply *mutatis mutandis* to the method for enhancing the potency of a thrombolytic agent for treating a GPVI-related condition in a subject in need thereof.

### BRIEF DESCRIPTION OF THE DRAWINGS

**Figure 1** is a schema representing the mRS results at Day 90 across treatment groups (glenzocimab at 1000 mg (n=52) vs placebo (n=53)) in the overall population.
**Figure 2** is a schema representing the mRS results at Day 90 across treatment groups (glenzocimab at 1000 mg (n=52) vs placebo (n=53)) in the subpopulation of patients with a Tan score of 2 or 3. No patient associated with a mRS of 5 is present in the groups of patients treated with glenzocimab or placebo.

### EXAMPLES

The present invention is further illustrated by the following examples.

### Example 1: Subpopulation with a large ischemic volume

### Materials and Methods

Patients from the clinical trial NCT03803007, who received glenzocimab (*i.e*. ACT017) or placebo, were sorted in three groups according to infarct volume at baseline. Small volume corresponds to the lower tertial, *i.e.* the lower third of acute infarct volume (AIV). Medium volume corresponds to the intermediate tertial, *i.e.* the median third of acute infarct volume (AIV). Large volume corresponds to the upper tertial, *i.e.* the upper third of acute infarct volume (AIV).

Acute Infarct Volume (AIV) is the estimate of ischemic core volume at baseline. It was used at baseline to define subgroups, and to compare with Follow-Up Infarct Volume to determine infarct growth and penumbral salvage. e-MRI was used to define AIV using an apparent diffusion coefficient (ADC) threshold of <620×10-6mm²s⁻¹. e-CTP used a relative cerebral blood flow (rCBF) threshold of <30% to define AIV.

Table 4 hereinbelow represents the three groups defined according to infarct volume at baseline.

**Table 4**

| Acute Infarct Volume (AIV) | Lower value (mL) | Upper value (mL) | Number of patients |
|---|---|---|---|
| Small | 0.0 | 0.5 | 55 |
| Medium | 0.6 | 6.8 | 54 |
| Large | 7.0 | 162.5 | 55 |

All the following parameters were calculated only for the phase 2a patients of the trial *(i.e.* patients receiving 1000 mg of glenzocimab or placebo).

The Follow-up Lesion/Infarct Volume (FIV) was measured at 24 hours in the general phase 2a population and in the groups of patients defined as hereinabove. Comparison of placebo versus glenzocimab 1000 mg was stratified according to the size of the ischemic lesion at baseline.

The FIV was determined through automated artificial intelligence quantification on CT, and these were adjusted if needed by expert imaging scientists and clinicians to generate the final biomarker. The FIV is a composite of the extent of the ischemic lesion (ISC) and any hemorrhagic transformation (HT), plus any contribution from anatomical distortion (AD) due to edema. As a result, the FIV could be corrected for AD, and also separated into the ISC and HT components. FIV was defined using NCCT at 24 hours.

The mortality was measured at 90 days in the general phase 2a population and in the groups of patients defined as hereinabove. Comparison of placebo versus glenzocimab 1000 mg was stratified according to the size of the ischemic lesion at baseline.

### Results

The FIV in the general phase 2a population and stratified according to the size of the ischemic lesion at baseline is presented in Table 5 hereinbelow.

**Table 5**

| | Treatment | Median ischemic volume at 24 hours (mL) | Interquartile range | Number of patients |
|---|---|---|---|---|
| All population | | | | |
| - | Glenzocimab | 12.0 | 31.6 | 53 |
| - | Placebo | 14.3 | 44.6 | 53 |
| Population stratified according to the size of the infarct volume at baseline | | | | |
| Medium | Glenzocimab | 8.2 | 23.9 | 20 |
| AIV | Placebo | 14.8 | 14.8 | 16 |
| Large AIV | Glenzocimab | 32.6 | 55.4 | 19 |
| | Placebo | 47.9 | 161.6 | 20 |

As disclosed herein, the median ischemic volume measured at 24 hours is 14.3 and 12.0 for the whole population treated with placebo and glenzocimab respectively, while the median ischemic volume is 47.9 and 32.6 for the subpopulations of patients with a large AIV treated with placebo and glenzocimab respectively. No statistically significant difference in median ischemic volume was observed at 24 hours for the subpopulations of patients with a low AIV treated with placebo and glenzocimab (data not shown).

The mortality measured at 90 days in the general phase 2a population and stratified according to the size of the ischemic lesion at baseline is presented in Table 6 hereinbelow.

**Table 6**

| | Treatment | Number of dead patients at day 90 | Percentage of deaths in the group |
|---|---|---|---|
| All population | | | |
| - | Glenzocimab | 4 | 7.5 |
| - | Placebo | 11 | 20.8 |
| Population stratified according to the size of the infarct volume at baseline | | | |
| Medium AIV | Glenzocimab | 1 | 5.0 |
| | Placebo | 3 | 18.8 |
| Large AIV | Glenzocimab | 2 | 10.5 |
| | Placebo | 7 | 35.0 |

As disclosed herein, the percentage of deaths is 20.8 and 7.5 for the whole population treated with placebo and glenzocimab respectively, while this percentage is 35.0 and 10.5 for the subpopulations of patients with a large AIV treated with placebo and glenzocimab respectively. No statistically significant difference in mortality was observed at 90 days for the subpopulations of patients with a low AIV treated with placebo and glenzocimab (data not shown).

Thus, these data show that glenzocimab (*i*.*e*. ACT017) is particularly effective when administered at 1000 mg as compared to the placebo, in the subpopulation of patients with large infarct volume (*i.e*. with an infarct volume equals or above 7.0 mL).

### Example 2: Subpopulation with good cerebral collateral circulation

### Materials and Methods

Patients from the clinical trial NCT03803007, who received glenzocimab (*i.e*. ACT017) or placebo, were sorted in two groups according to the Tan Score at baseline *(i.e.* one group with Tan score of 0 or 1 and one group with Tan score of 2 or 3).

The Tan Score was quantified with CTA. Automated quality control for phase of acquisition was also calculated.

The mRS was calculated only for the phase 2a patients of the trial (*i.e.* patients receiving 1000 mg of glenzocimab or placebo). The mRS was measured at day 90 after treatment. Comparison of placebo versus glenzocimab 1000 mg was stratified according to Tan score at baseline (groups 0-1 or 2-3).

### Results

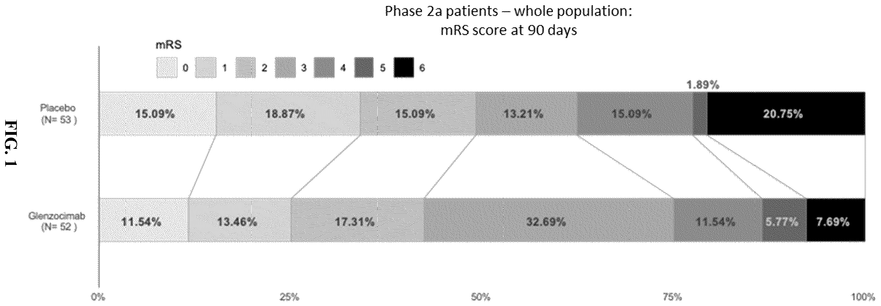

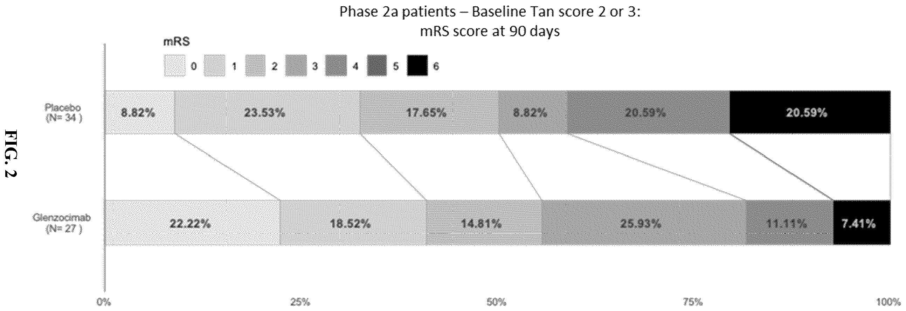

The mRS in the general phase 2a population and stratified according to Tan score at baseline is presented in Figures 1 and 2.

As shown in Figure 1, prevalence of moderately severe and severe disability was greater for patient treated with placebo, meaning that glenzocimab significantly reduced the number of patients showing a mRS ≥ 4 at 90 days. However, for the whole population, the number of patients in the lowest disability categories, *i.e.* with a mRS ≤ 2, is not significantly increased at 90 days in glenzocimab treated patients as compared to placebo receiving patients.

By contrast, as shown in Figure 2, the number of patients in the lowest disability categories, *i.e.* with a mRS ≤ 2, is increased at 90 days in glenzocimab treated patients with a Tan score of 2 or 3 at baseline, as compared to placebo receiving patients with a Tan score of 2 or 3 at baseline. These data illustrate that glenzocimab is able to reduce the proportion of patients with mild, moderate or severe disability in the subpopulation of patients with a Tan score of 2 or 3 at baseline.

Thus, these results show that glenzocimab (*i.e.* ACT017) is particularly effective when administered at 1000 mg as compared to the placebo, in the subpopulation of patients with a good collateral circulation (*i.e.* with a Tan score of 2-3).

## Claims

1. An isolated humanized protein binding to human Glycoprotein VI (hGPVI) for use in treating a GPVI-related condition in a subject in need thereof, wherein said subject presents at least one of the following conditions:
i) said subject has a large ischemic core volume, and/or
ii) said subject has a good cerebral collateral circulation,
wherein the cerebral collateral circulation is assessed by either measuring the Tan Score with computed tomography angiography (CTA), or measuring the hypoperfusion intensity ratio (HIR) with CTP or magnetic resonance imaging perfusion (MRP).

2. The isolated humanized protein binding to hGPVI for use according to claim 1, wherein the GPVI-related condition is a stroke, preferably an acute ischemic stroke.

3. The isolated humanized protein binding to hGPVI for use according to any one of claims **1** to **2**, wherein the ischemic core volume is assessed by measuring the acute infarct volume (AIV) with magnetic resonance imaging (MRI), and/or computed tomography (CT), including CT perfusion (CTP), non-contrast computed tomography (NCCT) and computed tomography angiography (CTA).

4. The isolated humanized protein binding to hGPVI for use according to any one of claims **1** to **3**, wherein the subject with a large ischemic core volume has an ischemic core volume equals or above 7.0 mL.

5. The isolated humanized protein binding to hGPVI for use according to any one of claims **1** to **4**, wherein the cerebral collateral circulation is assessed by either measuring the Tan Score with computed tomography angiography (CTA).

6. The isolated humanized protein binding to hGPVI for use according to any one of claims **1** to **5**, wherein the subject with a good cerebral collateral circulation has a Tan score value of 2 or 3.

7. The isolated humanized protein binding to hGPVI for use according to any one of claims **1** to **6**, wherein the protein protects the cerebral tissue following the GPVI-related condition.

8. The isolated humanized protein binding to hGPVI for use according to any one of claims **1** to **7**, wherein the protein decreases the volume of infarct, the volume of hemorrhagic transformation and/or the volume of ischemic injury following the GPVI-related condition.

9. The isolated humanized protein binding to hGPVI for use according to any one of claims **1** to **8**, being an antibody molecule or an antibody fragment selected from the group consisting of a whole antibody, a single chain variable fragment (scFv), a Fv fragment, a Fab fragment, a unibody, a domain antibody, and a nanobody; or a monomeric antibody mimetic selected from the group consisting of an affibody, an affilin, an affitin, an adnectin, an atrimer, an evasin, a DARPin, an anticalin, an avimer, a fynomer, and a versabody.

10. The isolated humanized protein binding to hGPVI for use according to any one of claims **1** to **9**, wherein the isolated humanized protein is a monovalent antibody or fragment thereof.

11. The isolated humanized protein binding to hGPVI for use according to any one of claims **1** to **10**, being an antibody molecule or an antibody fragment, wherein
- the variable region of the heavy chain comprises at least one of the following CDRs:
VH-CDR1: GYTFTSYNMH (SEQ ID NO: 1);
VH-CDR2: GIYPGNGDTSYNQKFQG (SEQ ID NO: 2); and
VH-CDR3: GTVVGDWYFDV (SEQ ID NO: 3);
or any CDR having an amino acid sequence that shares at least 60% of identity with SEQ ID NO: 1-3; and
- the variable region of the light chain comprises at least one of the following CDRs:
VL-CDR1: RSSQSLENSNGNTYLN (SEQ ID NO: 4);
VL-CDR2: RVSNRFS (SEQ ID NO: 5); and
VL-CDR3: LQLTHVPWT (SEQ ID NO: 6);
or any CDR having an amino acid sequence that shares at least 60% of identity with SEQ ID NO: 4-6.

12. The isolated humanized protein binding to hGPVI for use according to any one of claims **1** to **11**, being an antibody molecule or an antibody fragment, wherein the variable region of the heavy chain comprises the following CDRs: GYTFTSYNMH (SEQ ID NO: 1), GIYPGNGDTSYNQKFQG (SEQ ID NO: 2) and GTVVGDWYFDV (SEQ ID NO: 3) and the variable region of the light chain comprises the following CDRs: RSSQSLENSNGNTYLN (SEQ ID NO: 4), RVSNRFS (SEQ ID NO: 5) and LQLTHVPWT (SEQ ID NO: 6) or any CDR having an amino acid sequence that shares at least 60% of identity with said SEQ ID NO: 1-6.

13. The isolated humanized protein binding to hGPVI for use according to any one of claims **1** to **12**, being an antibody molecule or an antibody fragment, wherein the amino acid sequence of the heavy chain variable region is SEQ ID NO: 7 or any sequence having an amino acid sequence that shares at least 60% of identity with said SEQ ID NO: 7, and wherein the amino acid sequence of the light chain variable region is SEQ ID NO: 8, or any sequence having an amino acid sequence that shares at least 60% of identity with said SEQ ID NO: 8.

14. The isolated humanized protein binding to hGPVI for use according to any one of claims **1** to **10**, being an antibody molecule or an antibody fragment, wherein the amino acid sequence of the heavy chain variable region is SEQ ID NO: 7 or any sequence having an amino acid sequence that shares at least 60% of identity with said SEQ ID NO: 7, and wherein the amino acid sequence of the light chain variable region is SEQ ID NO: 9, or any sequence having an amino acid sequence that shares at least 60% of identity with said SEQ ID NO: 9.
